# EUROPÄISCHE PATENTSCHRIFT

(11) **EP 3 229 591 B1**
(45) Veröffentlichungstag und Bekanntmachung des Hinweises auf die Patenterteilung: **03.10.2018**
(21) Anmeldenummer: 15807936.8
(22) Anmeldetag: 10.12.2015
(51) Int. Cl.: A01N 25/30, A01N 41/10, C07H 15/12, C07C 233/20

(54) **ZUCKERTENSIDE UND DEREN VERWENDUNG IN AGROCHEMISCHEN ZUSAMMENSETZUNGEN**
SUGAR SURFACTANTS AND USE THEREOF IN AGROCHEMICAL COMPOSITIONS
TENSIOACTIFS DE SUCRE ET LEUR UTILISATION DANS DES COMPOSITIONS AGROCHIMIQUES

(30) Priorität: 12.12.2014 DE 102014018274
(43) Veröffentlichungstag der Anmeldung: 18.10.2017
(73) Patentinhaber: Clariant International Ltd, 4132 Muttenz (CH)
(72) Erfinder: KLUG, Peter, 63762 Grossostheim (DE); SCHERL, Franz-Xaver, 84508 Burgkirchen (DE); NUNES, George Italo Pitombeira, 4310 Rheinfelden (CH); BAUR, Peter, 86938 Schondorf (DE); ARNOLD, Roland, 65627 Elbtal (DE)
(74) Vertreter: Paczkowski, Marcus
(86) Internationale Anmeldenummer: PCT/EP2015/079295
(87) Internationale Veröffentlichungsnummer: WO 2016/092030

(56) Entgegenhaltungen:
- WO-A1-96/16540
- WO-A1-2014/067663

## Beschreibung

Die Erfindung betrifft Zuckertenside, eine wässrige Adjuvant-Zusammensetzung enthaltend die Zuckertenside, deren Verwendung in und zur Herstellung von agrochemischen Zusammensetzungen sowie wässrige, insbesondere agrochemische Zusammensetzungen enthaltend die erfindungsgemäßen Zuckertenside.

Pestizide (vor allem Fungizide, Herbizide und Insektizide) sind chemische Substanzen synthetisch hergestellt oder natürlichen Ursprungs, die in Pflanzenzellen, -gewebe oder in parasitäre Organismen in oder auf der Pflanze eindringen und diese schädigen und/oder zerstören. Den größten Anteil an Pestiziden stellen Herbizide dar. Pestizide werden üblicherweise in Form von flüssigen oder festen konzentrierten Zubereitungen (Formulierungen) in der Landwirtschaft eingesetzt. Die erleichtern dem Anwender so die Handhabung oder sorgen für eine höhere Wirksamkeit des Wirkstoffs. Die Formulierungen werden üblicherweise vor dem Einsatz mit Wasser verdünnt und anschließend durch Sprühapplikation ausgebracht.

Wasserlösliche Konzentrate (Soluble Liquids, abgekürzt mit SL) sind eine besonders wichtige Form der Pestizidzubereitungen. Sie spielen insbesondere bei Herbiziden eine große Rolle, wobei die Pestizide oftmals als wasserlösliche Salze, die durch Neutralisation der Säureform der Herbizide mit geeigneten Basen in ihre Alkali- oder Ammoniumsalze überführt werden, eingesetzt werden.

Eine besonders wichtige Rolle spielen die wasserlöslichen Salze von Herbiziden, wie beispielsweise des Glyphosats, Glufosinats oder der Auxin-Herbizide wie 2,4-D oder Dicamba. Sie werden vorzugsweise als Alkalimetallsalz oder in Form verschiedener Ammoniumsalze bzw. als Gemisch dieser Salze meistens als wässrige Formulierungen verwendet.

Ein generelles Problem bei der Anwendung von Pestiziden ist, dass nur ein Bruchteil des Wirkstoffes die gewünschte Aktivität entfaltet. Der größere Teil geht oft ungenutzt verloren, indem der Wirkstoff bei der Ausbringung der Spritzbrühe nicht die Blätter oder
die Wurzeln der Pflanze erreicht, sondern ungenutzt im Boden versickert, durch Regen abgewaschen oder von der Pflanze einfach nicht richtig aufgenommen wird.

Dieser ökologische und ökonomische Nachteil kann durch Zugabe von Hilfsstoffen, im Rahmen der vorliegenden Anmeldung als "Adjuvants" bezeichnet, zu Pestizid-Formulierungen reduziert werden. Diese Hilfsstoffe können beispielsweise den Spray-Drift reduzieren, die Benetzung der Pflanze verbessern oder dafür sorgen, dass der Wirkstoff länger auf der Pflanzenoberfläche haftet bzw. besser aufgenommen wird. Insbesondere bei wasserlöslichen Pestiziden, wie beispielsweise bei Glyphosat haben die Art sowie die Menge der verwendeten Adjuvants einen entscheidenden Einfluss auf die Wirksamkeit der Formulierung.

Die mit Abstand am häufigsten verwendeten Adjuvants in wässrigen Herbizid-Formulierungen sind Fettaminethoxylate, hauptsächlich Talgfettaminethoxylate. Diese Produkte sind jedoch aufgrund ihrer toxischen und ökotoxikologischen Eigenschaften, wie der starken Augenreizung oder der Toxizität gegenüber aquatischen Organismen als bedenklich einzustufen und werden zunehmend durch Adjuvants mit einem besseren toxikologischen und ökotoxikologischen Profil ersetzt.

Adjuvants, die in wässrigen Pestizidformulierungen eingesetzt werden, liegen üblicherweise in flüssiger Form, d. h. als wassermischbare Lösungen vor, um die Herstellung der Pestizidformulierung zu vereinfachen. Die Adjuvant-Lösungen können Wasser und/ oder wassermischbare Lösemittel enthalten, die zusammen mit dem Pestizid eine homogene und lagerstabile wässrige Formulierung ergeben. Wenn möglich wird Wasser als Lösemittel eingesetzt, da dies sowohl aus Kostenals auch aus Umweltgesichtspunkten bevorzugt ist. Gegebenenfalls werden Co-Solventien zugesetzt, die in der Lage sind, die Löslichkeit oder die Stabilität zu verbessern.

Die Verwendung von zuckerbasierenden Tensiden, wie Alkyl-N-methylglucosamiden, beispielsweise in Reinigungsmitteln und kosmetischen Produkten ist in der Literatur beschrieben (F.W. Lichtenthaler, "Carbohydrates as Organic Raw Materials" in Ullmann's Ullmann's Encyclopedia of Industrial Chemistry, Wiley-VCH Verlag, 2010).

WO 96/16540 beschreibt Pestizidzusammensetzungen, die langkettige Alkylamide, die einen Polyhydroxycarbonyl-Substituenten mit mindestens drei Hydroxylgruppen am Amidstickstoff tragen.

In der WO 2014/067663 sind wässrige Adjuvant-Zusammensetzungen beschrieben, die ein oder mehrere Alkylglucamide mit einer linearen oder verzweigten C₅-C₉-Alkylgruppe enthalten und sich zur Wirkungssteigerung von Pestiziden und zum Herstellen wässriger Pestizid-Zusammensetzungen eignen.

Die Anforderungen an Adjuvants in wässrigen Pestizid-Zusammensetzungen sind im Laufe der Jahre stetig angewachsen. Neben hoher biologischer Wirksamkeit und Unbedenklichkeit, sowohl aus Sicht des Anwenders als auch unter Umweltgesichtspunkten, werden zunehmend vorteilhaftere anwendungstechnische Eigenschaften gefordert. Die Adjuvants sollen eine möglichst hohe Beladung der Formulierung mit dem Wirkstoff ermöglichen und möglichst mit verschiedenen Wirkstoffen kompatibel sein. Die Formulierungen müssen lagerstabil sein und eine möglichst niedrige Viskosität aufweisen, um eine leichtere Handhabung zu gewährleisten, sowie die möglichst vollständige Entleerung der Gebinde erleichtern. Außerdem ist eine gute Mischbarkeit und schnelles Lösevermögen, auch und besonders in kaltem Wasser, beim Ansetzen der Spritzbrühe gefordert.

Es stellte sich somit die Aufgabe, neue wässrige Adjuvant-Zusammensetzungen zur Verfügung zu stellen, die hochwirksam sind, die sich durch ein sehr vorteilhaftes toxikologisches und ökologisches Profil auszeichnen und die aus anwendungstechnischer Sicht vorteilhafte Eigenschaften aufweisen.

Es wurde gefunden, dass sich Zuckertenside enthaltend einen 9-Decenoylrest in besonderer Weise zur Verwendung als Adjuvants in agrochemischen Zusammensetzungen eignen.

Gegenstand der Erfindung ist daher ein Zuckertensid der Formel (I), 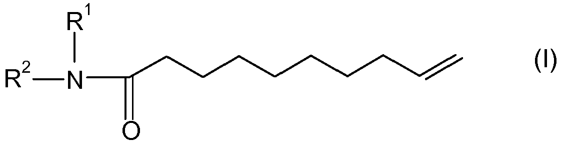 worin
- R¹: H oder eine Alkylgruppe mit 1 bis 3 Kohlenstoffatomen bedeutet und
- R²: eine Polyhydroxyhydrocarbyl-Gruppe mit einer linearen, bevorzugt C₄-C₆, Hydrocarbyl-Kette bedeutet, wobei mindestens drei Hydroxygruppen direkt an die Kette gebunden sind, bevorzugt eine 2,3,4,5,6-Pentahydroxyhex-1yl-Gruppe.

Weiterhin Gegenstand der Erfindung ist eine vorzugsweise einphasige Adjuvant-Zusammensetzung, enthaltend
A1) ein oder mehrere erfindungsgemäße Zuckertenside der Formel (I),
A2) Wasser,
A3) gegebenenfalls ein Co-Solvens und
A4) gegebenenfalls ein oder mehrere Ammoniumsalze
A5) gegebenenfalls einen oder mehrere Enthärter.

Weiterhin Gegenstand der Erfindung ist die Verwendung von erfindungsgemäßen Zuckertensiden und der erfindungsgemäßen Zusammensetzung als Adjuvants für agrochemische Wirkstoff-Zusammensetzungen.

Weiterhin Gegenstand der Erfindung ist eine Wirkstoff-Zusammensetzung, insbesondere agrochemische Wirkstoff-Zusammensetzung enthaltend das erfindungsgemäße Zuckertensid, insbesondere die erfindungsgemäße Adjuvant-Zusammensetzung.

Ebenso Gegenstand der Erfindung ist ein Verfahren zur Bekämpfung von Schadorganismen, wobei man den Schadorganismus oder dessen Lebensraum mit einer agrochemischen Wirkstoff-Zusammensetzung, enthaltend als agrochemischen Wirkstoff mindestens ein Pestizid und die erfindungsgemäße Adjuvant-Zusammensetzung, in Kontakt bringt.

Das erfindungsgemäße Zuckertensid und die erfindungsgemäße Adjuvant-Zusammensetzung erhöhen insbesondere die Wirksamkeit von ElektrolytWirkstoffen, insbesondere Pestiziden und Pflanzennährstoffen, die als wasserlösliche Salze vorliegen.

### Zuckertensid

Die Gruppe R² wird vorzugsweise aus einem reduzierenden Zucker durch reduktive Aminierung erhalten. Geeignete reduzierende Zucker sind beispielsweise Glucose, Fructose, Maltose, Lactose, Galactose, Mannose und Xylose. Als Ausgangsmaterial eignet sich neben den genannten Zuckern auch Maissirup mit hohem Dextrose-, Fructose- oder Maltosegehalt (High Dextrose Corn Syrup, High Fructose Corn Syrup, High Maltose Corn Syrup). Aus diesen Maissiruptypen ergeben sich insbesondere Mischungen verschiedener Zuckerkomponenten für die Zuckertenside der Formel (I).

In den Zuckertensiden der Formel (I) steht R¹ bevorzugt für eine C₁-C₃-Alkylgruppe, besonders bevorzugt für Methyl.
- R²: ist bevorzugt -CH₂-(CHOH)ₙ-CH₂OH, -CH₂-(CH₂OH)(CHOH)ₙ₋₁-CH₂OH, -CH₂-(CHOH)₂(CHOR³)(CHOH)-CH₂OH, wobei
- n: 3, 4 oder 5, bevorzugt 4, ist und
- R³: H oder ein cyclisches Mono- oder Polysaccharid ist.
- R²: ist besonders bevorzugt -CH₂-(CHOH)₄-CH₂OH.
Ganz besonders bevorzugt als Zuckertensid (I) ist N-Methyl-N-dec-9-enoyl-glucamin (la) 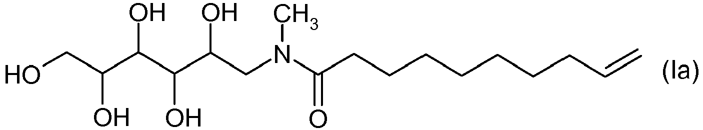 Dabei ist der Zuckerrest R² ganz besonders bevorzugt von D-Glucose abgeleitet.
Die Herstellung der erfindungsgemäßen Zuckertenside (I) erfolgt beispielsweise durch Umsetzung der entsprechenden aus reduktiven Zuckern erhältlichen Zuckeramine, wie N-Methylglucamin, mit dem ebenfalls kommerziell erhältlichen 9-Decensäuremethylester. Ein entsprechendes Herstellungsverfahren ist beispielsweise in der EP-A 0 550 637 beschrieben.

Offenbart ist auch ein Verfahren zur Herstellung von Zuckertensiden der Formel (I), wobei man ein Zuckeramin der Formel (II),

R¹R²NH (II)

wobei R¹ und R² die in der Formel (I) angegebenen Bedeutungen haben, mit 9-Decensäuremethylester, vorzugsweise in einem protischen Lösungsmittel wie 1,2-Propylenglykol, umsetzt.

### Adjuvant-Zusammensetzung

Erfindungsgemäße Adjuvant-Zusammensetzungen weisen neben mindestens einem Zuckertensid (I) (A1) und Wasser (A2) gegebenenfalls ein Co-Solvens (A3) und gegebenenfalls ein oder mehrere Ammoniumsalze (A4) und gegebenenfalls einen oder mehrere Enthärter (A5) auf.
Der Anteil der erfindungsgemäßen Zuckertenside (I) (Komponente (A1)) in der Adjuvant-Zusammensetzung beträgt bevorzugt 10 bis 90 Gew.-%, besonders bevorzugt 20 bis 80 Gew.-% und insbesondere bevorzugt 30 bis 70 Gew.-%, bezogen auf das Gesamtgewicht der Zusammensetzung. In einer weiteren Ausführungsform der Erfindung beträgt der Anteil der erfindungsgemäßen Zuckertenside (I) (Komponente (A1)) in der Adjuvant-Zusammensetzung bevorzugt 5 bis 90 Gew.-%, besonders bevorzugt 10 bis 80 Gew.-% und insbesondere bevorzugt 10 bis 30 Gew.-%.
Als Komponente (A2) enthalten die Adjuvant-Zusammensetzungen Wasser. Als Wasser kommen beispielsweise entsalztes Wasser, Grund-, See- oder Leitungswasser in Frage. Bevorzugt hat das Wasser eine Härte von unter 15 ° dH (Deutscher Härte).
Der Wassergehalt beträgt bevorzugt 20 bis 89 Gew.-%, besonders bevorzugt 25 bis 70 Gew.-%, insbesondere bevorzugt 30 bis 60 Gew.-%.

Das optional enthaltene Co-Solvens (A3) kann entweder als Nebenkomponente aus dem Herstellungsprozess des Alkylglucamids zugegen sein oder nachträglich zur Adjuvant-Zusammensetzung zugegeben worden sein. Bei dem Co-Solvens kann es sich um ein einziges Lösemittel oder ein Gemisch zweier oder mehrerer Lösemittel handeln. Dazu eignen sich alle polaren Lösemittel, die mit der wässrigen Zusammensetzung kompatibel sind und eine homogene Phase bilden. Geeignete Co-Solventien sind beispielsweise einwertige Alkohole, wie Methanol, Ethanol, Propanole, Butanole, Benzylalkohol oder mehrwertige Alkohole wie Ethylenglykol, Diethylenglykol, Propylenglykol oder Glycerin oder Polyglykole wie Polyethylen-, Polypropylen- oder gemischte Polyalkylenglykole (PAGs). Weitere geeignete Lösemittel sind Ether wie beispielsweise Propylenglykolmono- oder dimethylether, Dipropylenglykolmono- oder dimethylether, Amide wie beispielsweise N-Methyl- oder N-Ethylpyrrolidon, Milchsäure-, Capryl- oder Decansäuredimethylamid.

Besonders geeignete Co-Solventien sind ein oder mehrwertige Alkohole und insbesondere geeignet sind zwei- oder dreiwertige Alkohole wie Propylenglykol, Glycerin oder Polyethylen-, Polypropylen- bzw. gemischte Polyalkylenglykole (PAGs).

Ganz besonders bevorzugt enthalten die Adjuvant-Zusammensetzungen als Komponente (A3) Propylenglykol oder Dipropylenglykol, sowie Kombinationen beider untereinander oder mit Polypropylenglykol oder Polyethylenglykol mit bis zu 10 Wiederholendeinheiten. Insbesondere bevorzugt ist Propylenglykol.

Der Gehalt an Propylenglykol beträgt - soweit vorhanden - bevorzugt 1 bis 30 Gew.-%, besonders bevorzugt 2 bis 10 Gew.-%, insbesondere bevorzugt 2 bis 5 Gew.-%.

Der Anteil des Co-Solvens in der Zusammensetzung beträgt - soweit vorhanden - üblicherweise 10 bis 250 g/l, bevorzugt 20 bis 200 g/l und besonders bevorzugt 30 bis 150 g/l.

Das Co-Solvens kann zusätzlich zur Stabilisierung der Adjuvant-Zusammensetzungen beitragen, indem es beispielsweise die Kälte- oder Wärmestabilität erhöht oder weitere anwendungstechnische Eigenschaften wie die Viskosität positiv beeinflusst.

Als Komponente (A4) enthalten die Adjuvant-Zusammensetzungen gegebenenfalls ein oder mehrere, bevorzugt ein oder zwei, besonders bevorzugt ein wasserlösliches Ammoniumsalz.

Bevorzugt als Ammoniumsalze sind Ammoniumsulfat, Ammoniumnitrat, Ammoniumnitratharnstoff, Ammoniumphosphat, Ammoniumcitrat, Ammoniumthiosulfat und/oder Ammoniumchlorid, besonders bevorzugt Ammoniumsulfat, Ammoniumnitrat, Ammoniumcitrat und/oder Ammoniumnitratharnstoff, ganz besonders bevorzugt ist Ammoniumsulfat.

Falls die Komponente (A4) vorhanden ist, enthalten die Adjuvant-Zusammensetzungen bevorzugt 5 bis 60 Gew.-%, besonders bevorzugt 10 bis 50 Gew.-%, insbesondere bevorzugt 20 bis 50 Gew.-% des einen oder der mehreren Ammoniumsalze.

Als Komponente (A5) enthalten die erfindungsgemäßen Adjuvant-Zusammensetzungen gegebenenfalls einen Enthärter. Unter "Enthärter" wird erfindungsgemäß eine Substanz verstanden, die im Wasser gelöste Erdalkalikationen wie Ca²⁺ und Mg²⁺ beseitigt oder durch Komplexbildung maskiert.

Bevorzugt eingesetzte Enthärter sind Alkaliphosphate, Alkalicitrate und Alkalisulfate wie Na₂SO₄, Glycerinsulfate, Phosphonate, Nitrilotriacetat, Aminopolycarbonsäuren wie EDTA und DTPA, Aminopolyphosphonsäuren wie EDTMP und HEDP, Gluconate, Monocarbamid Dihydrogensulfat, Polycarboxylate wie Polyarylate und Polyphosphonate.

In einer bevorzugten Ausführungsform enthält die erfindungsgemäße Adjuvant-Zusammensetzung einen oder mehrere Enthärter.

In einer weiteren Ausführungsform enthält die erfindungsgemäße Adjuvant-Zusammensetzung keinen Enthärter.

In einer weiteren Ausführungsform können die erfindungsgemäßen Adjuvant-Zusammensetzungen ein oder mehrere weitere Hilfsstoffe (A6) enthalten, wobei es sich dabei beispielsweise um Konservierungsmittel, Tenside, Entschäumer, funktionelle Polymere oder zusätzliche Adjuvants handeln kann. Beispiele für Hilfsstoffe finden sich weiter unten.

In einer bevorzugten Ausführungsform bestehen die Adjuvant-Zusammensetzungen aus den Komponenten (A1) bis (A6), besonders bevorzugt (A1) bis (A5).

Die wässrigen Adjuvant-Zusammensetzungen der Zuckertenside der Formel (I) eignen sich als Adjuvants insbesondere in wässrigen agrochemischen Wirkstoff-Zusammensetzungen zur Verbesserung der biologischen Aktivität von Pestiziden wie Herbiziden, Insektiziden, Fungiziden, Akariziden, Bakteriziden, Molluskiden, Nematiziden und Rodentiziden sowie Pflanzennährstoffen und Pflanzenstärkungsmittel.

Gegenstand der Erfindung ist daher auch die Verwendung der wässrigen Adjuvant-Zusammensetzungen zur Steigerung der biologischen Aktivität von agrochemischen Wirkstoffen, insbesondere von Herbiziden.

Die wässrigen Adjuvant-Zusammensetzungen eignen sich hervorragend zur Herstellung von lagerstabilen agrochemischen Wirkstoff-Zusammensetzungen, die vorteilhafte Eigenschaften besitzen.

Gegenstand der Erfindung ist daher auch die Verwendung der erfindungsgemäßen Zusammensetzungen zur Herstellung von wässrigen agrochemischen Wirkstoff-Zusammensetzungen. Übliche Verfahren zur Herstellung solcher Zusammensetzungen sind dem Fachmann bekannt.

Gegenstand der Erfindung sind weiterhin wässrige Wirkstoff-Zusammensetzungen enthaltend
(W1) ein oder mehrere erfindungsgemäße Zuckertenside (I),
(W2) Wasser,
(W3) einen oder mehrere wasserlösliche Wirkstoffe, insbesondere agrochemische Wirkstoffe,
(W4) gegebenenfalls ein Co-Solvens
(W5) gegebenenfalls ein oder mehrere Ammoniumsalze,
(W6) gegebenenfalls einen oder mehrere Enthärter,
(W7) gegebenenfalls einen oder mehrere Hilfsstoffe
(W8) gegebenenfalls einen oder mehrere wasserunlösliche Wirkstoffe, insbesondere Pestizide.

Für die Komponente (W1), Zuckertensid, gilt analog das zu der Komponente (A1) der Adjuvant-Zusammensetzung Gesagte.

Für die Komponente (W2), Wasser, gilt analog das zu der Komponente (A2) der Adjuvant-Zusammensetzung Gesagte.

Als Wirkstoffe (W3) kommen neben den bevorzugten agrochemischen Wirkstoffen beispielsweise pharmazeutische und kosmetische Wirkstoffe in Betracht.

Unter wasserlöslichen agrochemischen Wirkstoffen (W3) im Sinne der Erfindung sind agrochemische Wirkstoffe zu verstehen, die bei Raumtemperatur (25 °C) eine Löslichkeit von mehr 50 g/l und vorzugsweise mehr als 100 g/l in Wasser aufweisen. Besonders bevorzugt sind agrochemische Wirkstoffe, die bei der Einsatzkonzentration bei 20 °C eine Löslichkeit von mindestens 90 Gew.-% aufweisen.

Als agrochemische Wirkstoffe seien bevorzugt Pestizide, Pflanzennährstoffe und Pflanzenstimulantien genannt, besonders bevorzugt Herbizide, Isektizide, Akarizide, Nematizide, Repellents, Fungizide, Bakterizide, Pflanzenwachstumsregulatoren, Pflanzennährstoffe und Pflanzenstärkungsmittel.

Die erfindungsgemäßen Zusammensetzungen sind insbesondere geeignet für Kombinationen mit einem oder mehreren der folgenden wasserlöslichen Wirkstoffe:
Acifluorfen, Aminopyralid, Amitrol, Asulam, Benazolin, Bentazon, Bialaphos, Bispyribac, Bromacil, Bromoxynil, Bicyclopyron, Chloramben, Clopyralid, 2,4-D, 2,4-DB, Dicamba, Dichlorprop, Difenzoquat, Diquat, Endothal, Fenoxaprop, Flamprop, Florasulam, Flumiclorac, Fluoroglycofen, Fluroxypyr, Fomesafen, Fosamine, Glufosinat, Glyphosat, Imizameth, Imazamethabenz, Imazamox, Imazapic, Imazapyr, Imazaquin, Imazethapyr, MCPA, MCPB, Mecoprop, Mesotrione, Nicosulfuron, Octansäure, Pelargonsäure, Picloram, Quizalofop, 2,3,6-TBA, Sulcotrione, Tembotrione und Triclopyr, besonders bevorzugt 2,4-D, Bentazon, Dicamba, Fomesafen, Glyphosat, Glufosinat, MCPA, Mesotrione Paraquat und Sulcotrione, bevorzugt aus den wasserlöslichen Salzen von Glyphosat und Dicamba.

Bevorzugte Salze sind dabei z.B. Acifluorfen-Natrium, Bialafos-Natrium, Bispyridac-Natrium, Glufosinat-Ammonium, Glufosinat-P-Ammonium, Glufosinat-P-Natrium, Glyphosat-Isopropylammonium, Glyphosat-Trimesium, Imazamox-Ammonium, Imazapyr-Isopropylammonium, Imazaquinammonium, Imazethapyr-Ammonium, MCPB-Natrium, Mecocrop-Natrium, Mecocrop-P-Dimethylammonium und Mecocrop-P-kalium.

Bevorzugt als wasserlösliche agrochemische Wirkstoffe sind weiterhin Pflanzennährstoffe, insbesondere anorganische, kationische Nährstoffe wie Eisen, Magnesium und Kalium und anorganische, kationische Mikronährstoffe wie Bor, Kupfer, Mangan, Molybdän und Zink, wobei Nährstoffe und Mikronährstoffe als Salze und/oder Chelatverbindungen vorliegen.

Ebenfalls bevorzugt sind Pflanzenwuchsregulatoren, beispielsweise natürliche Pflanzenhormone, insbesondere Abszissinsäure, Jasmonsäure, Salicylsäure und deren Ester, Kinetin und Brassinosteroide.

Weiterhin bevorzugt als Pflanzenstimulantien seien Substanzen genannt, die als Pflanzenstärkungsmittel wirken, um den Einfluss von Stressfaktoren wie Hitze, Kälte, Trockenheit, Salz, Sauerstoffmangel oder Überschwemmung auf das Pflanzenwachstum zu mindern. Beispiele sind Glycinbetain (Betain), Cholin, Phosphatsalze wie Kaliumphosphat sowie Silikate.

Die erfindungsgemäßen Zusammensetzungen können daneben, in der Formulierung oder auch der Spritzbrühe, weitere wasserunlösliche Wirkstoffe (W8) enthalten, die beispielsweise dispergiert vorliegen können.

Im Folgenden werden weitere Beispiele für agrochemische Wirkstoffe genannt, die gelöst als Komponente (W3) vorliegen oder Kombinationspartner dieser Pestizide (Komponente (W8)) bilden können.

Wirkstoffe, die auf einer Inhibition von beispielsweise Acetolaktat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441 445 oder "The Pesticide Manual", 16th edition, The British Crop Protection Council and the Royal Soc. of Chemistry, 2012 und dort zitierter Literatur beschrieben sind. Als bekannte Herbizide oder Pflanzenwachstumsregulatoren, die mit den erfindungsgemäßen Verbindungen kombiniert werden können, sind z. B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Aclonifen, Alachlor, Allidochlor, Alloxydim, Alloxydim-sodium, Ametryn, Amicarbazone, Amidochlor, Amidosulfuron, Aminocyclopyrachlor, Aminocyclopyrachlorkalium, Aminocyclopyrachlormethyl, Ammoniumsulfamat, Ancymidol, Anilofos, Atrazine, Aviglycin, Azafenidin, Azimsulfuron, Aziprotryn, Beflubutamid, Benazolinethyl, Bencarbazone, Benfluralin, Benfuresate, Bensulide, Bensulfuron, Bensulfuronmethyl, Benzfendizone, Benzobicyclon, Benzofenap, Benzofluor, Benzoylprop, Benzyladenin, Bifenox, Bilanafos, Bilanafosnatrium, Bromobutide, Bromofenoxim, Bromuron, Buminafos, Busoxinone, Butachlor, Butafenacil, Butamifos, Butenachlor, Butralin, Butroxydim, Butylate, Cafenstrole, Carbaryl, Carbetamide, Carfentrazone, Carfentrazoneethyl, Carvone, Chlorcholinchlorid, Chlomethoxyfen, Chlorazifop, Chlorazifopbutyl, Chlorbromuron, Chlorbufam, Chlorfenac, Chlorfenacnatrium, Chlorfenprop, Chlorflurenol, Chlorflurenol-methyl, Chloridazon, Chlorimuron, Chlorimuronethyl, Chlormequatchlorid, Chlornitrofen, 4-Chlorophenoxyacetic acid, Chlorophthalim, Chlorpropham, Chlorthaldimethyl, Chlorotoluron, Chlorsulfuron, Cinidon, Cinidonethyl, Cinmethylin, Cinosulfuron, Clethodim, Clodinafop, Clodinafoppropargyl, Clofencet, Clomazone, Clomeprop, Cloprop, Cloransulam, Cloransulammethyl, Cloxyfonac, Cumyluron, Cyanamide, Cyanazine, Cyclanilide, Cycloate, Cyclosulfamuron, Cycloxydim, Cycluron, Cyhalofop, Cyhalofopbutyl, Cyperquat, Cyprazine, Cyprazole, Cytokinine, Daimuron/Dymron, Dalapon, Daminozide, Dazomet, n-Decanol, Desmedipham, Desmetryn, Detosyl-Pyrazolate (DTP), Diallate, Diaminozid, Dichlobenil, Dichlorprop-P, Diclofop, Diclofopmethyl, Diclofop-P-methyl, Diclosulam, Diethatyl, Diethatylethyl, Difenoxuron, Diflufenican, Diflufenzopyr, Diflufenzopyrnatrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Diisopropylnaphthalene, Dipropetryn, Diquatdibromide, Dithiopyr, Diuron, DNOC, Eglinazineethyl, EPTC, Esprocarb, Ethalfluralin, Ethametsulfuron, Ethametsulfuronmethyl, Ethylnaphthylacetat, Ethephon, Ethidimuron, Ethiozin, Ethofumesate, Ethoxyfen, Ethoxyfenethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl)pyrimidin-2,4(1H,3H)-dion, Fenoprop, Fenoxapropethyl, Fenoxaprop-P-ethyl, Fenoxasulfone, Fentrazamide, Fenuron, Flamprop, Flamprop-M-isopropyl, Flamprop-M-methyl, Flazasulfuron, Fluazifop, Fluazifop-P, Fluazifopbutyl, Fluazifop-P-butyl, Fluazolate, Flucarbazone, Flucarbazonesodium, Flucetosulfuron, Fluchloralin, Flufenacet (Thiafluamide), Flufenpyr, Flufenpyrethyl, Flumetralin, Flumetsulam, Flumicloracpentyl, Flumioxazin, Flumipropyn, Fluometuron, Fluorodifen, Fluoroglycofenethyl, Flupoxam, Flupropacil, Flupropanate, Flupyrsulfuron, Flupyrsulfuronmethylsodium, Flurenol, Flurenolbutyl, Fluridone, Flurochloridone, Fluroxypyr-meptyl, Flurprimidol, Flurtamone, Fluthiacet, Fluthiacetmethyl, Fluthiamide, Foramsulfuron, Forchlorfenuron, Furyloxyfen, Gibberellinsäure, H-9201, d. h. O-(2,4-Dimethyl-6-nitrophenyl)-O-ethylisopropylphosphor amidothioat, Halosafen, Halosulfuron, Halosulfuronmethyl, Haloxyfop, Haloxyfop-P, Haloxyfopethoxyethyl, Haloxyfop-P-ethoxyethyl, Haloxyfopmethyl, Haloxyfop-P-methyl, Hexazinone, HW-02, d. h. 1-(Dimethoxyphosphoryl)-ethyl-(2,4-dichlorphenoxy)acetat, Imazamethabenzmethyl, Imazosulfuron, Inabenfide, Indanofan, Indaziflam, Indolessigsäure (IAA), 4-Indol-3-ylbuttersäure (IBA), lodosulfuron, lodosulfuronmethyl-natrium, lofensulfuron, lofensulfuronnatrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPB-methyl, -ethyl, Mecopropbutotyl, Mecoprop-P-butotyl, Mecoprop-P-2-ethylhexyl, Mefenacet, Mefluidide, Mepiquat-chlorid, Mesosulfuron, Mesosulfuronmethyl, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuronmethyl, Molinate, Monalide, Monocarbamide, Monocarbamidedihydrogensulfat, Monolinuron, Monosulfuron, Monosulfuronester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, 1-Naphtylacetic Acid (NAA), Naphthylacetamid (NAAm), 2-Naphtoxyacetic Acid, Naproanilide, Napropamide, Naptalam, NC-310, d.h. 4-(2,4-Dichlorobenzoyl)-1-methyl-5-benzyloxypyrazole, Neburon, Nipyraclofen, Nitralin, Nitrofen, Nitroguaiacolate, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquatdichlorid, Pendimethalin, Pendralin, Penoxsulam, Pentanochlor, Pentoxazone, Perfluidone, Pethoxamid, Phenisopham, Phenmedipham, Phenmediphamethyl, Picolinafen, Pinoxaden, Piperophos, Pirifenop, Pirifenopbutyl, Pretilachlor, Primisulfuron, Primisulfuronmethyl, Probenazole, Profluazol, Procyazine, Prodiamine, Prifluraline, Profoxydim, Prohexadione, Prohexadionecalcium, Prohydrojasmone, Prometon, Prometryn, Propachlor, Propanil, Propaquizafop, Propazine, Propham, Propisochlor, Propoxycarbazone, Propoxycarbazonenatrium, Propyrisulfuron, Propyzamide, Prosulfalin, Prosulfocarb, Prosulfuron, Prynachlor, Pyraclonil, Pyraflufen, Pyraflufenethyl, Pyrasulfotole, Pyrazolynate (Pyrazolate), Pyrazosulfuron, Pyrazosulfuronethyl, Pyrazoxyfen, Pyribambenz, Pyribambenzisopropyl, Pyribambenzpropyl, Pyribenzoxim, Pyributicarb, Pyridafol, Pyridate, Pyriftalid, Pyriminobac, Pyriminobac-methyl, Pyrimisulfan, Pyrithiobac, Pyrithiobacnatrium, Pyroxasulfone, Pyroxsulam, Quinclorac, Quinmerac, Quinoclamine, Quizalofopethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulfallate (CDEC), Sulfentrazone, Sulfometuron, Sulfometuron-methyl, Sulfo-sulfuron, SW-065, SYN-523, SYP-249, d.h. 1-Ethoxy-3-methyl-1-oxobut-3-en-2-yl-5-[2-chlor-4-(trifluormethyl)phenoxy]-2-nitrobenzoat, SYP-300, d.h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazonemethyl, Thifensulfuron, Thifensulfuronmethyl, Thiobencarb, Tiocarbazil, Topramezone, Tralkoxydim, Triafamone, Triallate, Triasulfuron, Triaziflam, Triazofenamide, Tribenuron, Tribenuronmethyl, Tribufos, Trichloressigsäure (TCA), Tridiphane, Trietazine, Trifloxysulfuron, Trifloxysulfuronnatrium, Trifluralin, Triflusulfuron, Triflusulfuronmethyl, Trimeturon, Trinexapac, Trinexapacethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

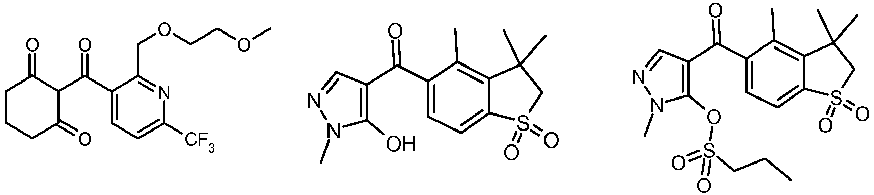

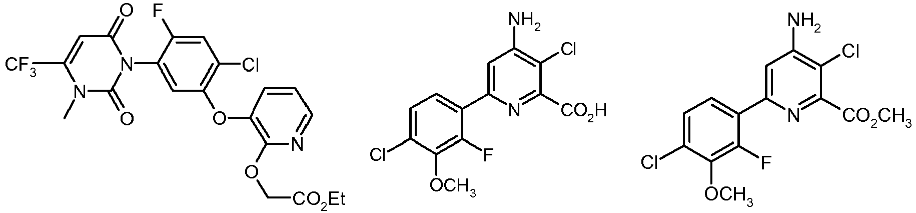

Als weitere Beispiele für Pflanzennährstoffe seien übliche anorganische oder organische Dünger zur Versorgung von Pflanzen mit Makro- und/oder Mikronährstoffen genannt.

Als Beispiele für Fungizide seien genannt:
(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dode-morph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3-dihydro-1H-inden-1-yl)-1H-imidazol-5-carboxylat, N'-{5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des synepimeren Razemates 1RS,4SR,9RS und des antiempimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (synepimeres Razemat 1RS,4SR,9RS), Isopyrazam (synepimeres Enantiomer 1R,4S,9R), Iso-pyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1-methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid, N-[1-(2,4-Dichlorphenyl)-1-methoxypropan-2-yl]-3-(difluormethyl)-1-methyl-1H-pyrazol-4-carboxamid, 5,8-Difluor-N-[2-(2-fluor-4-{[4-(trifluormethyl)pyridin-2-yl]oxy}phenyl)ethyl]quinazolin-4-amin, N-[9-(Dichlormethylen)-1,2,3,4-tetrahydro-1,4-methanonaphthalen-5-yl]-3-(difluormethyl)-1-methyl-1H-pyrazol-4-carboxamid, N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid.
(3) Inhibitoren der Respiration (Atmungsketten-Inhibitoren) am Komplex III der Atmungskette, wie beispielsweise Ametoctradin, Amisulbrom, Azoxystrobin, Cyazofamid, Coumethoxystrobin, Coumoxystrobin, Dimoxystrobin, Enestroburin, Famoxadon, Fenamidon, Fenoxystrobin, Fluoxastrobin, Kresoxim-Methyl, Metominostrobin, Orysastrobin, Picoxystrobin, Pyraclostrobin, Pyrametostrobin, Pyraoxystrobin, Pyribencarb, Triclopyricarb, Trifloxystrobin, (2E)-2-(2-{[6-(3-Chlor-2-methylphenoxy)-5-fluorpyrimidin-4-yl]oxy}phenyl)-2-(methoxyimino)-N-methylethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]-amino}oxy)methyl]phenyl}-2-(methoxyimino)-N-methylethanamid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, Methyl-(2E)-2-{2-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethyl-phenoxy)methyl]phenyl}-2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfe-nazol, Diethofencarb, Ethaboxam, Fluopicolid, Fuberidazol, Pencycuron, Thiabendazol, Thi-ophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasti-cidin-S, Cyprodinil, Kasugamycin, Kasugamycin Hydrochlorid Hydrat, Mepanipyrim, Pyri-methanil und 3-(5-Fluor-3,3,4,4-tetramethyl-3,4-dihydroisoquinolin-1-yl)quinolin.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flu-morph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon, Tricyclazol und 2,2,2-Trifluorethyl {3-methyl-1-[(4-methylbenzoyl)amino]butan-2-yl}carbamat.
(12) Inhibitoren der Nukleinsäuresynthese, wie beispielsweise Benalaxyl, Benalaxyl-M (Kirala-xyl), Bupirimat, Clozylacon, Dimethirimol, Ethirimol, Furalaxyl, Hymexazol, Metalaxyl, Metalaxyl-M (Mefenoxam), Ofurace, Oxadixyl und Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Pyriofenon (Chlazafenon), Cufraneb, Cyflufenamid, Cymoxanil, Cyprosulfa-mide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrimorph, (2E)-3-(4-Tert-butylphenyl)-3-(2-chlorpyridin-4-yl)-1-(morpholin-4-yl)prop-2-en-1-on, (2Z)-3-(4-Tert-butylphenyl)-3-(2-chlorpyridin-4-yl)-1-(morpholin-4-yl)prop-2-en-1-on, Pyrrolnitrin, Tebufloquin, Tecloftalam, Tolnifanid, Triazoxid, Trichlamid, Zarilamid, (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2,6-Dimethyl-1H,5H-[1,4]dithiino[2,3-c:5,6-c']dipyrrol-1,3,5,7(2H,6H)-tetron, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1 ,2-oxazol-3-yl)-1 ,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1H-imidazol-5-yl]pyridin, 2-Phenylphenol und dessen Salze, 3-(4,4,5-Trifluor-3,3-dimethyl-3,4-dihydroisoquinolin-1-yl)quinolin, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Fluor-2-[(4-fluorbenzyl)oxy]pyrimidin-4-amin, 5-Fluor-2-[(4-methylbenzyl)oxy]pyrimidin-4-amin, 5-Methyl-6-octyl[1,2,4]triazolo[1,5-a]pyrimidin-7-amin, Ethyl-(2Z)-3-amino-2-cyan-3-phenylprop-2-enoat, N'-(4-{[3-(4-Chlorbenzyl)-1,2,4-thiadiazol-5-yl]oxy}-2,5-dimethylphenyl)-N-ethyl-N-methylimidoformamid, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropyl-methoxy)¬imino][6-(difluormethoxy)-2,3-difluorphenyl]methyl}-2-phenylacetamid, N'-{4-[(3-Tert-butyl-4-cyano-1,2-thiazol-5-yl)oxy]-2-chlor-5-methylphenyl}-N-ethyl-N-methylimidoformamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}¬piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaph¬thalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol, Chinolin-8-olsulfat(2:1) und Tert-butyl {6-[({[(1-methyl-1H-tetrazol-5-yl)(phenyl)methylen]amino}oxy)methyl]pyridin-2-yl}carbamat.
(16) Weitere Verbindungen, wie beispielsweise 1-Methyl-3-(trifluormethyl)-N-[2'-(trifluormethyl)biphenyl-2-yl]-1H-pyrazol-4-carboxamid, N-(4'-Chlorbiphenyl-2-yl)-3-(difluormethyl)-1-methyl-1H-pyrazol-4-carboxamid, N-(2',4'-Dichlorbiphenyl-2-yl)-3-(difluormethyl)-1-methyl-1H-pyrazol-4-carboxamid, 3-(Difluormethyl)-1-methyl-N-[4'-(trifluormethyl)biphenyl-2-yl]-1H-pyrazol-4-carboxamid, N-(2',5'-Difluorbiphenyl-2-yl)-1-methyl-3-(trifluormethyl)-1H-pyrazol-4-carboxamid, 3-(Difluormethyl)-1-methyl-N-[4'-(prop-1-in-1-yl)biphenyl-2-yl]-1H-pyrazol-4-carboxamid, 5-Fluor-1,3-dimethyl-N-[4'-(prop-1-in-1-yl)biphenyl-2-yl]-1H-pyrazol-4-carboxamid, 2-Chlor-N-[4'-(prop-1-in-1-yl)biphenyl-2-yl]pyridin-3-carboxamid, 3-(Difluormethyl)-N-[4'-(3,3-dimethylbut-1-in-1-yl)biphenyl-2-yl]-1-methyl-1H-pyrazol-4-carboxamid, N-[4'-(3,3-Dimethylbut-1-in-1-yl)biphenyl-2-yl]-5-fluor-1,3-dimethyl-1H-pyrazol-4-carboxamid, 3-(Difluormethyl)-N-(4'-ethinylbiphenyl-2-yl)-1-methyl-1H-pyrazol-4-carboxamid, N-(4'-Ethinylbiphenyl-2-yl)-5-fluor-1,3-dimethyl-1H-pyrazol-4-carboxamid, 2-Chlor-N-(4'-ethinylbiphenyl-2-yl)pyridin-3-carboxamid, 2-Chlor-N-[4'-(3,3-dimethylbut-1-in-1-yl)biphenyl-2-yl]pyridin-3-carboxamid, 4-(Difluormethyl)-2-methyl-N-[4'-(trifluormethyl)biphenyl-2-yl]-1,3-thiazol-5-carboxamid, 5-Fluor-N-[4'-(3-hydroxy-3-methylbut-1-in-1-yl)biphenyl-2-yl]-1,3-dime¬thyl-1H-pyrazol-4-carboxamid, 2-Chlor-N-[4'-(3-hydroxy-3-methylbut-1-in-1-yl)biphenyl-2-yl]pyridin-3-carboxamid, 3-(Difluormethyl)-N-[4'-(3-methoxy-3-methylbut-1-in-1-yl)biphenyl-2-yl]-1-methyl-1H-pyrazol-4-carboxamid, 5-Fluor-N-[4'-(3-methoxy-3-methylbut-1-in-1-yl)biphenyl-2-yl]-1,3-dimethyl-1H-pyrazol-4-carboxamid, 2-Chlor-N-[4'-(3-methoxy-3-methylbut-1-in-1-yl)biphenyl-2-yl]pyridin-3-carboxamid, (5-Brom-2-methoxy-4-methylpyridin-3-yl)(2,3,4-trimethoxy-6-methylphenyl)methanon, N-[2-(4-{[3-(4-Chlorphenyl)prop-2-in-1-yl]oxy}-3-methoxyphenyl)ethyl]-N2-(methylsulfonyl)valin-amid, 4-Oxo-4-[(2-phenylethyl)amino]butansäure und But-3-yn-1-yl {6-[({[(Z)-(1-methyl-1H-tetrazol-5-yl)(phenyl)methylen]amino}oxy)methyl]pyridin-2-yl}carbamat.

Alle genannten Fungizide (1) bis (16) können, wenn sie aufgrund ihrer funktionellen Gruppen dazu imstande sind, gegebenenfalls mit geeigneten Basen oder Säuren Salze bilden.

Als Beispiele für Bakterizide seien genannt:
Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

Als Beispiele für Insektizide, Akarizide und Nematizide seien genannt:
(1) Acetylcholinesterase (AChE) Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder Organophosphate, z. B. Acephate, Azamethiphos, Azinphosethyl, Azinphosmethyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphosmethyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodienorganochlorine, z. B. Chlordane und Endosulfan; oder Phenylpyrazole (Fiprole), z. B. Ethiprole und Fipronil.
(3) Natrium-Kanal-Modulatoren / Spannungsabhängige Natrium-Kanal-Blocker, wie beispielsweise Pyrethroide, z. B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-lsomer), Prallethrin, Pyrethrine (pyrethrum), Resmethrin, Silafluofen, Tefluthrin, Tetramethrin, Tetramethrin [(1R)-Isomere)], Tralomethrin und Transfluthrin; oder DDT; oder Methoxychlor.
(4) Nikotinerge Acetylcholin-Rezeptor (nAChR) Agonisten, wie beispielsweise Neonikotinoide, z. B. Acetamiprid, Clothianidin, Dinotefuran, Imidacloprid, Nitenpyram, Thiacloprid und Thiamethoxam; oder Nikotin.
(5) Nikotinerge Acetylcholin-Rezeptor (nAChR) allosterische Aktivatoren, wie beispielsweise Spinosine, z. B. Spinetoram und Spinosad.
(6) Chlorid-Kanal-Aktivatoren, wie beispielsweise Avermectine/Milbemycine, z. B. Abamectin, Emamectin-benzoat, Lepimectin und Milbemectin.
(7) Juvenilhormon-Imitatoren, wie beispielsweise Juvenilhormon-Analoge, z. B. Hydroprene, Kinoprene und Methoprene; oder Fenoxycarb; oder Pyriproxyfen.
(8) Wirkstoffe mit unbekannten oder nicht spezifischen Wirkmechanismen, wie beispielsweise Alkylhalide, z. B. Methylbromid und andere Alkylhalide; oder Chloropicrin; oder Sulfurylfluorid; oder Borax; oder Brechweinstein.
(9) Selektive Fraßhemmer, z. B. Pymetrozine; oder Flonicamid.
(10) Milbenwachstumsinhibitoren, z. B. Clofentezine, Hexythiazox und Diflovidazin; oder Etoxazole.
(11) Mikrobielle Disruptoren der Insektendarmmembran, z. B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder Organozinnverbindungen, z. B. Azocyclotin, Cyhexatin und Fenbutatin-oxid; oder Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise METI-Akarizide, z. B. Fenazaquin, Fenpyroximate, Pyrimidifen, Pyridaben, Tebufenpyrad und Tolfenpyrad; oder Rotenone (Derris).
(22) Spannungsabhängige Natriumkanal-Blocker, z. B. Indoxacarb; oder Metaflumizone.
(23) Inhibitoren der Acetyl-CoA-Carboxylase, beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z. B. Aluminiumphosphid, Calciumphosphid, Phosphin und Zinkphosphid; oder Cyanid.
(25) Komplex-II-Elektronentransportinhibitoren, beispielsweise Cyenopyrafen.
(26) Ryanodinrezeptor-Effektoren, wie beispielsweise Diamide, z. B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Pyridalyl, Pyrifluquinazon und lodmethan; desweiteren Präparate auf Basis von Bacillus firmus (I-1582, BioNeem, Votivo) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), 4-{[(6-Chlorpyrid-3-yl)methyl](2,2-difluorethyl)amino}furan-2(5H)-on (bekannt aus WO2007/115644), 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ4-sulfanyliden}cyanamid (bekannt aus WO2007/149134) und seine Diastereomere {[(1R)-1-(6-Chlorpyridin-3-yl)ethyl](methyl)oxido-λ4-sulfanyliden}cyanamid (A) und {[(1S)-1-(6-Chlorpyridin-3-yl)ethyl](methyl)oxido-λ4-sulfanyliden}cyanamid (B) (ebenfalls bekannt aus WO2007/149134) sowie Sulfoxaflor (ebenfalls bekannt aus WO2007/149134) und seine Diastereomere [(R)-Methyl(oxido){(1R)-1-[6-(trifluormethyl)pyridin-3-yl]ethyl}-λ4-sulfanyliden]¬cyanamid (A1) und [(S)-Methyl(oxido){(1S)-1-[6-(trifluormethyl)pyridin-3-yl]ethyl}-λ4-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ4-sulfanyliden]cyanamid (B1) und [(S)-Methyl(oxido){(1R)-1-[6-(trifluormethyl)pyridin-3-yl]ethyl}-λ4-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt aus WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), 2-Ethyl-7-methoxy-3-methyl-6-[(2,2,3,3-tetrafluor-2,3-dihydro-1,4-benzodioxin-6-yl)oxy]chinolin-4-yl-methylcarbonat (bekannt aus JP2008/110953), 2-Ethyl-7-methoxy-3-methyl-6-[(2,2,3,3-tetrafluor-2,3-dihydro-1,4-benzodioxin-6-yl)oxy]chinolin-4-ylacetat (bekannt aus JP2008/110953), PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl]¬(2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002/096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002/096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluor-pyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502) und (1E)-N-[(6-Chlorpyridin-3-yl)methyl]-N'-cyan-N-(2,2-difluorethyl)ethanimidamid (bekannt aus WO2008/009360).

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides).

Bei den agrochemischen Wirkstoffen Komponente (W3) und/oder (W8) kann es sich auch um eine Kombination von zwei oder von mehreren agrochemischen Wirkstoffen handeln. Solche Kombinationen sind insbesondere dann von Bedeutung, wenn es beispielsweise darum geht, das Wirkungsspektrum der agrochemischen Wirkstoff-Zusammensetzung zu verbreitern oder Resistenzen gegenüber bestimmten agrochemischen Wirkstoffen besser zu unterbinden.

Die Kombination von zwei oder mehreren agrochemischen Wirkstoffen in einer Formulierung ist ein schwieriges Unterfangen. Die Wirkstoffe sind üblicherweise nicht miteinander kompatibel und die wässrigen Mischungen daher nicht phasenstabil. Die erfindungsgemäßen Adjuvant-Zusammensetzungen eignen sich aber gut dazu, um solche grundsätzlich inkompatiblen Zusammensetzungen zu stabilisieren.

In einer weiteren Ausführungsform der Erfindung enthalten die erfindungsgemäßen agrochemischen Wirkstoff-Zusammensetzungen daher mindestens zwei wasserlösliche agrochemische Wirkstoffe der Komponente (W3).

Bevorzugt sind die mindestens zwei wasserlöslichen agrochemischen Wirkstoffe Herbizide, ausgewählt aus Glyphosat, Glufosinat, 2,4-D, Dicamba und Fomesafen.

Besonders bevorzugte Zusammensetzungen sind dabei solche, bei denen es sich bei den wasserlöslichen agrochemischen Wirkstoffen der Komponente (W3) um die Kombinationen der zwei Herbizide Glyphosat und 2,4-D, Glyphosat und Dicamba, Glyphosat und Fomesafen, Glyfosat und Glufosinat, 2,4-D und Dicamba, Glufosinat und 2,4-D sowie Glufosinat und Dicamba handelt.

Bei der Formulierung von wässrigen agrochemischen Wirkstoff-Zusammensetzungen ist man bestrebt, die Zusammensetzung mit einer möglichst hohen Konzentration an Wirkstoff zu beladen. Das reduziert Verpackungs-, Transport-, Lager- und Entsorgungskosten. Daher sollte eine Adjuvant-Zusammensetzung in der Lage sein, stabile hochbeladene agrochemische Wirkstoff-Zusammensetzungen, sogenannte "High-Load-Formulierungen", zu ermöglichen. Dies gelingt mit den Alkylglucamiden der Formel (I) überraschend gut.

In einer bevorzugten Ausführungsform der Erfindung beträgt die Menge des einen oder der mehreren wasserlöslichen agrochemischen Wirkstoffe der Komponente (W3) in den erfindungsgemäßen Zusammensetzungen mehr als 100 g/l, bevorzugt mehr als 200 g/l und besonders bevorzugt mehr als 300 g/l. Diese Mengenangaben beziehen sich auf das Gesamtgewicht der erfindungsgemäßen agrochemischen Wirkstoff-Zusammensetzung und im Falle von agrochemischen Wirkstoffen, die in Form ihrer wasserlöslichen Salze eingesetzt werden (wie üblicherweise beispielsweise Glyphosat oder 2,4-D), auf die Menge an freier Säure, dem sogenannten Säureäquivalent ("acid equivalent", a.e.).

In einer weiteren bevorzugten Ausführungsform der Erfindung beträgt die Menge des einen oder der mehreren Zuckertenside der Formel (I) in den erfindungsgemäßen agrochemischen Wirkstoff-Zusammensetzungen 20 bis 250 g/l, bevorzugt 40 bis 200 g/l und besonders bevorzugt 50 bis 150 g/l. Diese Mengenangaben beziehen sich auf die gesamte Menge der erfindungsgemäßen agrochemischen Wirkstoff-Zusammensetzung.

Üblicherweise werden die Zuckertenside der Formel (I) in Form von Lösungen eingesetzt. Zur Klarstellung sei hier erwähnt, dass sich die oben genannten Mengenangaben dabei auf den Aktivgehalt der Zuckertenside der Formel (I) in der Lösung beziehen.

Ein besonders wichtiges Kriterium für die Lagerstabilität von wässrigen agrochemischen Wirkstoff-Zusammensetzungen wie Glyphosat- und 2,4-D-Formulierungen, ist die Phasenstabilität. Eine Zusammensetzung wird dann als ausreichend phasenstabil angesehen, wenn sie über einen weiten Temperaturbereich homogen bleibt und wenn es nicht zur Ausbildung von zwei oder mehreren getrennten Phasen oder zu Ausfällungen (Bildung einer weiteren festen Phase) kommt. Phasenstabilität ist sowohl bei erhöhter Temperatur, wie sie beispielsweise bei der Lagerung in der Sonne oder in warmen Ländern auftreten kann, als auch bei niedriger Temperatur, wie beispielsweise im Winter oder in kalten klimatischen Regionen, die entscheidende Voraussetzung für eine lagerstabile Formulierung.

Die erfindungsgemäßen agrochemischen Wirkstoff-Zusammensetzungen zeichnen sich dadurch aus, dass sie auch bei einer Temperatur von vorzugsweise größer 55 °C, besonders bevorzugt von größer 70 °C und insbesondere bevorzugt von größer 80 °C phasenstabil sind.

Außerdem zeichnen sich die erfindungsgemäßen agrochemischen Wirkstoff-Zusammensetzungen dadurch aus, dass sie auch bei einer Temperatur von vorzugsweise kleiner 10 °C, besonders bevorzugt von kleiner 0 °C und insbesondere bevorzugt von kleiner -10 °C phasenstabil sind.

Der pH-Wert der agrochemischen Wirkstoff-Zusammensetzungen liegt üblicherweise im Bereich von 3,5 bis 8,0, bevorzugt bei 4,0 bis 7,0 und besonders bevorzugt bei 4,5 bis 6,5 (gemessen als 1 Gew.-%ige wässrige Verdünnung). Der pH-Wert wird primär bestimmt durch die pH-Werte der Lösungen der wässrigen Pestizide, die als Salze schwacher Säuren vorliegen. Durch Zugabe von Säuren oder Basen kann der pH-Wert auf einen anderen Wert abweichend von dem ursprünglichen pH-Wert der Mischung eingestellt werden.

Die hohe Salzstabilität der erfindungsgemäßen agrochemischen Wirkstoff-Zusammensetzung im wässrigen Medium auch bei hoher Wirkstoff- und Salzkonzentration stellt einen großen anwendungstechnischen Vorteil dar. Sie ermöglicht es auch, agrochemische Salze wie beispielsweise Dünger in die Zusammensetzung aufzunehmen.

Gegebenenfalls enthalten die erfindungsgemäßen Wirkstoff-Zusammensetzungen ein oder mehrere Co-Solventien (Komponente (W4)).

In einer bevorzugten Ausführungsform enthalten die Wirkstoff-Zusammensetzungen mindestens ein Co-Solvens. In einer weiteren Ausführungsform enthalten die Wirkstoff-Zusammensetzungen kein Co-Solvens.

Für die Komponente (W4) gilt analog das zu der Komponente (A3) der Adjuvant-Zusammensetzung Gesagte.

Gegebenenfalls enthalten die erfindungsgemäßen Wirkstoff-Zusammensetzungen ein oder mehrere Ammoniumsalze (Komponente (W5)).

In einer bevorzugten Ausführungsform enthalten die Wirkstoff-Zusammensetzungen mindestens ein Ammoniumsalz. In einer weiteren Ausführungsform enthalten die Wirkstoff-Zusammensetzungen kein Ammoniumsalz.

Für die Komponente (W5) gilt analog das zu der Komponente (A4) der Adjuvant-Zusammensetzung Gesagte.

Gegebenenfalls enthalten die erfindungsgemäßen Wirkstoff-Zusammensetzungen ein oder mehrere Enthärter (Komponente (W6).

In einer bevorzugten Ausführungsform enthalten die Wirkstoff-Zusammensetzungen mindestens einen Enthärter. In einer weiteren Ausführungsform enthalten die Wirkstoff-Zusammensetzungen keinen Enthärter.

Für die Komponente (W6) gilt analog das zu der Komponente (A5) der Adjuvant-Zusammensetzung Gesagte.

Die erfindungsgemäßen agrochemischen Wirkstoff-Zusammensetzungen enthalten gegebenenfalls ein oder mehrere weitere Hilfsstoffe (W7), wobei es sich bei diesen beispielsweise um Konservierungsmittel, weitere, von dem Zuckertensid (I) verschiedene Tenside, Entschäumer, funktionelle Polymere oder zusätzliche Adjuvants handeln kann.

Als Konservierungsmittel können organische Säuren und ihre Ester, beispielsweise Ascorbinsäure, Ascorbinpalmitat, Sorbat, Benzoesäure, Methyl- und Propyl-4-hydroxybenzoat, Propionate, Phenol, beispielsweise 2-Phenylphenat, 1,2-Benzisothiazolin-3-on, Formaldehyd, schwefelige Säure und deren Salze eingesetzt werden.

Bei den Tensiden kann es sich generell um alle mit der Zusammensetzung kompatiblen nichtionischen, amphoteren, kationischen oder anionische Tenside handeln.

Beispiele für nicht-ionische Tenside sind Ethoxylate und Alkoxylate von längerkettigen aliphastischen oder aromatischen Alkoholen, Fettaminethoxylate, längerkettige Etheraminalkoxylate, (gegebenenfalls ethoxylierte) Sorbitanester, Alkylpolyglycoside. Geeignete amphotere Tenside sind u.a. langkettige Alkyldimethylbetaine oder Alkyldimethylaminoxide, oder Alkyldimethylaminamidopropylaminoxide. Unter den anionischen Tensiden sind beispielsweise Ethersulfate von ethoxylierten Fettalkoholen, Umsetzungsprodukte von (gegebenenfalls ethoxylierten) langkettigen Alkoholen mit Phosphorsäurederivaten geeignet. Unter langkettig werden lineare oder verzweigte Kohlenwasserstoffketten mit mindestens 6 und maximal 22 Kohlenstoffatomen geeignet.

In einer bevorzugten Ausführungsform enthält die Wirkstoff-Zusammensetzung neben dem Zuckertensid der Formel (I) keine weiteren Zuckertenside.

Als Entschäumer eignen sich Fettsäurealkylesteralkoxylate, Organopolysiloxane wie Polydimethylsiloxane und deren Gemische mit mikrofeiner, gegebenenfalls silanierter Kieselsäure; Perfluoralkylphosphonate und -phosphinate, Paraffine, Wachse und Mikrokristallinwachse und deren Gemische mit silanierter Kieselsäure. Vorteilhaft sind auch Gemische verschiedener Schauminhibitoren, beispielsweise solche aus Silikonöl, Paraffinöl und/oder Wachsen.

Bei den funktionellen Polymeren, die in der erfindungsgemäßen Pestizid-Zusammensetzung enthalten sein können, handelt es sich hochmolekulare Verbindungen synthetischen oder natürlichen Ursprungs mit einer Molmasse von größer als 10.000. Die funktionellen Polymere können beispielsweise als Anti-Drift-Agent wirken oder die Regenfestigkeit steigern.

In einer weiteren bevorzugten Ausführungsform der Erfindung enthalten die erfindungsgemäßen agrochemischen Wirkstoff-Zusammensetzungen als Komponente (W7) ein oder mehrere weitere Adjuvants, wie sie bekanntermaßen in wässrigen Pestizid-Zusammensetzungen verwendet werden können.

Bevorzugt sind dies Fettaminethoxylate, Etheraminethoxylate, Alkylbetaine oder Amidoalkylbetaine, Aminoxide oder Amidoalkylaminoxide, Alkylpolylglycoside oder Copolymere aus Glycerin, Kokosfettsäure und Phthalsäure.

Diese Adjuvants sind aus der Literatur als Adjuvants in wässrigen Pestizid-Zusammensetzungen bekannt und beispielsweise in der WO2009/029561 beschrieben.

In einer weiteren bevorzugten Ausführungsform der Erfindung liegen die erfindungsgemäßen agrochemischen Wirkstoff-Zusammensetzungen als Konzentrat-Formulierungen vor, die vor dem Gebrauch verdünnt werden, insbesondere mit Wasser (beispielsweise "ready-to-use"-, "in-can"- oder "built-in"-Formulierungen), und enthalten das eine oder die mehreren wasserlöslichen agrochemischen Wirkstoffe der Komponente (W3) im Allgemeinen in Mengen von 5 bis 80 Gew.-%, bevorzugt von 10 bis 70 Gew.-% und besonders bevorzugt von 20 bis 60 Gew.-%, das eine oder die mehreren Zuckertenside der Formel (I) (W1) in Mengen von 1 bis 25 Gew.-, bevorzugt von 2 bis 20 Gew.-%, besonders bevorzugt von 3 bis 15 Gew.-%, das Ammoniumsalz (W5) - falls vorhanden - in Mengen von 5 bis 50 Gew.-%, bevorzugt 10 bis 40 Gew.-%, besonders bevorzugt 10 bis 35 Gew.-%, Co-Solvent (W4) in Mengen von 1 bis 30 Gew.-%, bevorzugt von 2 bis 10 Gew.-%, besonders bevorzugt 2 bis 5 Gew.-% und - falls vorhanden - Enthärter (W6) in Mengen von 1 bis 50 Gew.-%, bevorzugt 2 bis 25 Gew.-%, besonders bevorzugt 3 bis 10 Gew.-%.. Diese Mengenangaben beziehen sich auf die gesamte Konzentrat-Formulierung und im Falle von Pestiziden, die in Form ihrer wasserlöslichen Salze eingesetzt werden, auf die Menge an freier Säure, dem sogenannten Säureäquivalent ("acid equivalent", a.e.).

Die erfindungsgemäßen Pestizid-Zusammensetzungen werden vorzugsweise in Form von Spritzbrühen auf die Felder ausgebracht. Dabei werden die Spritzbrühen durch Verdünnung von Konzentrat-Formulierungen mit einer definierten Menge Wasser hergestellt.

In einer weiteren bevorzugten Ausführungsform der Erfindung liegen die erfindungsgemäßen agrochemischen Wirkstoff-Zusammensetzungen als Spritzbrühen vor und enthalten 0,001 bis 10 Gew.-%, bevorzugt 0,02 bis 3 Gew.-% und besonders bevorzugt 0,025 bis 2 Gew.-% des einen oder der mehreren wasserlöslichen agrochemischen Wirkstoffe der Komponente (W3) und 0,001 bis 3 Gew.-%, bevorzugt 0,005 bis 1 Gew.-% und besonders bevorzugt 0,01 bis 0,5 Gew.-% des einen oder der mehreren Zuckertenside der Formel (I) (W1). Die genannten Mengenangaben beziehen sich auf die gesamte Spritzbrühe und im Falle von agrochemischen Wirkstoffen, die in Form ihrer wasserlöslichen Salze eingesetzt werden, auf die Menge an freier Säure, dem sogenannten Säureäquivalent ("acid equivalent", a.e.).

Die Erfindung betrifft weiterhin die Verwendung erfindungsgemäßer agrochemischer Wirkstoff-Zusammensetzungen, bei denen der Wirkstoff ein Pestizid ist, zur Kontrolle und/oder zur Bekämpfung von unerwünschtem Pflanzenwuchs, Pilzerkrankungen oder Insektenbefall. Bevorzugt ist die Verwendung solcher Pestizid-Zusammensetzungen zur Kontrolle und/oder zur Bekämpfung von unerwünschtem Pflanzenwuchs.

Diese Verwendungen können vorzugsweise auch im sogenannten Tank-mix-Verfahren stattfinden. Hierbei können also das oder die mehreren wasserlöslichen agrochemischen Wirkstoffe der Komponente (W3) und das eine oder die mehreren Zuckertenside der Formel (I) sowie das Wasser auch in Form einer sogenannten "Tank-mix"- Zubereitung vorliegen. In einer derartigen Zubereitung liegen sowohl das oder die mehreren wasserlöslichen agrochemischen Wirkstoffe als auch das eine oder die mehreren Zuckertenside der Formel (I), letztere gegebenenfalls zusammen mit weiteren Adjuvants, getrennt voneinander vor. Beide Zubereitungen werden vor dem Ausbringen, in der Regel kurz vorher, miteinander vermischt, wobei eine erfindungsgemäße agrochemische Wirkstoff-Zusammensetzung entsteht.

### Ausführungsbeispiele

Im Folgenden wird die Erfindung anhand von Beispielen für den Fachmann noch weiter verdeutlicht.

### Beispiel 1: Herstellung einer erfindungsgemäßen wässrigen Zuckertensid-Zusammensetzung

Die Lösung mit 50 % Aktivsubstanz N-Methyl-N-dec-9-enoyl-glucamin wurde folgendermaßen hergestellt: Zunächst wurde 9-Decensäure (Aldrich) mit einen Überschuss Methanol zum 9-Decensäuremethylester umgesetzt. Der 9-Decensäuremethylester wurde destilliert und anschließend mit N-Methylglucamin in Gegenwart von 1,2-Propylenglykol als Lösungsmittel nach EP 0 550 637 umgesetzt und als Feststoff bestehend aus 90 % Aktivsubstanz und 10 % 1,2-Propylenglykol erhalten. Dieser Feststoff wurde bei 40 bis 50 °C in Wasser gelöst, so dass sich eine Lösung mit 50 % Gehalt an N-Methyl-N-dec-9-enoyl-glucamin ergab. Es handelt sich um eine klare, farblose Lösung.

### Beispiel 2

### Steigerung der Aufnahme systemischer Wirkstoffe am Beispiel Mesotrione und Testsystem zur Messung der Penetrationsförderung von Wirkstoffen

Tenside können die Aufnahme von (Wirk-)Stoffen durch Membranen wie Haut, Folien oder die pflanzliche Kutikula fördern. Als sogenannte "Finite-dose"-Applikation ist für die einmalige Applikation oder Auftragung einer Lösung, Creme, Gel etc. auf eine Membran bekannt, dass die Wirkstoffaufnahme auch nach erfolgter Benetzung durch manche Zusatzstoffe wie Tenside beeinflussbar ist. Dieser Effekt ist unabhängig von der Grenzflächenwirkung in Wasser, oft stark konzentrationsabhängig und findet größtenteils nach Verflüchtigung von Wasser und eventuell anwesenden Lösungsmitteln statt als Folge der Wechselwirkung z. B. mit Wirkstoff, Membran und Umweltfaktoren. Für verschiedene Tenside wird nach Zusatz zu Wirkstoffzubereitungen beobachtet, dass die Penetration eines bestimmten Wirkstoffes durch manche Tenside enorm gefördert wird während andere völlig unwirksam sind (Cronfeld, P, Lader, K. Baur, P. (2001). Classification of Adjuvants and Adjuvant Blends by Effects on Cuticular Penetration, Pesticide Formulations and Application Systems: Twentieth Volume, ASTM STP 1400, A. K. Viets, R. S. Tann, J. C. Mueninghoff, Edsl, American Society for Testing and Materials, West Conshohocken, PA 2001).

Das von der Tensidwirkung unabhängige Potential der Testsubstanzen, die Blattaufnahme von agrochemischen Wirkstoffen zu fördern wurde in Membranpenetrations-Versuchen mit Blattkutikeln von Apfel für Mesotrione bestimmt. Mesotrione ist der wichtigste moderne Herbizid-Wirkstoff und wie andere bedeutsame Herbizide (etwa 2,4-D oder Dicamba) eine schwache organische Säure mit einem pKa Wert von 3.1 im Bereich 2-6 und hat gutes systemisches Verhalten nach Aufnahme über die Blattkutikula. Mit einer Reihe solcher Säuren bzw. Elektrolytwirkstoffen wurde bereits eine durchgehend ausgezeichnete Aufnahmeförderung mit Synergen GA (N-Methyl-N-Octanoyl/Decanoyl-glucamin) insbesondere zusammen mit Ammoniumsulfat bei Verwendung von Wasser mit für saure Herbizide potentiell antagonistischen Gehalten an Erdalkali wie Calciumionen gefunden. Die pflanzliche Kutikula stellt eine lipophile Löslichkeitsmembran (Lipidmembran) ohne Poren oder Löcher dar und die beschrieben Ergebnisse werden mit diesen oder anderen Elektrolytwirkstoffen auch für andere nicht poröse lipophile Löslichkeitsmembranen erwartet. Das Prinzip der Methode ist veröffentlicht (z. B. WO-A-2005/194844; Baur, 1997; Baur, Grayson und Schönherr 1999; Baur, Bodelon und Lowe, 2012) und nur die Spezifika und methodische Abweichungen werden nachfolgend erklärt. Die Blattkutikeln wurden auf die in der Literatur beschriebene Weise von Apfelblättern von Feldbäumen einer kommerziellen Kernobstanlage in der Nähe von Frankfurt am Main im Jahr 2011 enzymatisch isoliert. Die zunächst an der Luft getrockneten stomatafreien Kutikeln wurden in Diffusionszellen aus Edelstahl eingebaut. Nach Applikation auf die ursprüngliche Blattoberseite und Verdunstung der Testflüssigkeit, d. h. der wässrigen Zubereitungen der Wirkstoffe ohne oder mit den Glucamid-haltigen Spritzflüssigkeiten oder Vergleichsmitteln wurden die Diffusionszellen in thermostatisierte Blöcke überführt und mit wässriger Flüssigkeit gefüllt. Als Wasser zum Ansetzen der wässrigen Testflüssigkeiten wurde lokales Leitungswasser (bekannter Zusammensetzung) verwendet. In regelmäßigen Abständen wurden Aliquot-Proben genommen und per HPLC der penetrierte Wirkstoffanteil bestimmt. Während des Versuchs waren die Temperatur im System (Block, Diffusionszellen, Flüssigkeiten etc.) und die Luftfeuchtigkeit über dem Spritzbelag auf der Kutikula exakt bekannt und kontrolliert. Im Versuch wurde die relative Luftfeuchte durchgehend konstant bei 56% relativer Luftfeuchte (Luft über übersättigtem Calciumnitrat) konstant bei 25°C. Die analytische Bestimmung mittels HPLC (1290 Infinity, Agilent) erfolgte nach mit Kinetex Säule 30x2, 1mm, 2.6µ C18 100A (Phenomenex) mit 20µl Aliquotnahme als Injektionsvolumen zu den angegeben Zeiten. Es werden jeweils die geometrischen Mittelwerte der Penetration für intakte Membranen zu den mittleren Messzeiten gegeben. Je Variante (Wirkstoff x Testadditiv/-formulierung) wurden 7-8 Wiederholungen angesetzt. Der Variationskoeffizient lag unter 35%, was für zahlreiche Pflanzen eine typische biologische Variabilität für die Penetration ist (Baur, 1997).

**Tabelle 1 Penetration von Mesotrione (0.3 g/l Wirkstoffkonzentration in Spritzflüssigkeit) in Anwesenheit von 0.75 g/l Ammoniumsulfat und der Glucamide Synergen GA (N-Methyl-N-Octanoyl/Decanoyl-glucamin), N-Methyl-N-dec-9-enoyl-glucamin und N-Methyl-N-nonanoyl-glucamin (alle mit 50% Aktivsubstanz)**

| Testprodukt (Konz.) | Konzentration Spritzbrühe [%] | Mittlere Penetration in % nach Zeit (n = 4-8) 0.3 g/l ai | |
|---|---|---|---|
| | | 12 - 14 Std. | 1 Tag |
| Synergen GA | 0.1 | 3.4 | 5.8 |
| Synergen GA | 0.25 | 4.4 | 7.1 |
| Synergen GA | 0.5 | 3.9 | 8.2 |
| N-Methyl-N-dec-9-enoyl-glucamin | 0.1 | 3.5 | 7.3 |
| N-Methyl-N-dec-9-enoyl-glucamin | 0.25 | 6.1 | 15.0 |
| N-Methyl-N-dec-9-enoyl-glucamin | 0.5 | 6.0 | 12.0 |
| N-Methyl-N-nonanoyl-glucamin | 0.1 | 3.1 | 6.7 |
| N-Methyl-N-nonanoyl-glucamin | 0.25 | 2.3 | 4.4 |
| N-Methyl-N-nonanoyl-glucamin | 0.5 | 6.7 | 11.5 |

| | | | |
|---|---|---|---|
| * 25 °C/56 % rel. Luftfeuchte | | | |

Die Tabelle zeigt, dass das N-Methyl-N-dec-9-enoyl-glucamin im Vergleich zum linearen Gemisch von C8 und C10 Glucamid zu einer höheren Penetrationsförderung von Mesotrione führte. Das ebenfalls mitgetestete N-Methyl-N-nonanoylglucamin fällt ebenso dahinter zurück.

### Beispiel 3 Dynamische Oberflächenspannung (Grenzflächenaktivität)

Bei schwer benetzbaren Pflanzen wie den Getreidepflanzen Weizen, Gerste, Triticale, Roggen und Hafer, bei weiteren großen Flächenkulturen Mais, Reis, Soja und Raps, wie auch bei fast allen Ungräsern und zahlreichen schwierig zu kontrollierenden dikotylen Unkräutern wie *Chenopodium album* oder *Euphorbium heterophyllum* ist die Förderung der Anlagerung der Spritzflüssigkeit auf den grünen Pflanzenteilen von entscheidender Bedeutung. Dieser Netzmitteleffekt wurde deshalb auch für die Glucamide bestimmt.

Für gegebene Applikationstechnik bzw. Parameter (Düse, Druck, Wasseraufwand, Anstand zur Pflanzenoberfläche) korreliert der Wert der dynamischen Oberflächenspannung in [mN/m] gut mit der Haftung auf schwer benetzbaren Pflanzen wie Gerste (bzw. allgemein Getreide) und Ungräsern. Ein Wert von 50 mN/m (bei 20-21 °C) ergibt gegenüber Wasser (72.8 mN/m) eine Verbesserung der Haftung von "Null Haftung" auf etwa 50 % (Baur P, Pontzen R 2007. Basic features of plant surface wettability and deposit formation and the impact of adjuvants. In: R E Gaskin ed. Proceeding of the 8th International Symposium on Adjuvants for Agrochemicals. Publisher: International Society for Agrochemical Adjuvants (ISAA), Columbus, Ohio, USA). Ein Wert von unter 60 mN/m bei 200 ms gibt eine sichtbar bessere Anlagerung wässriger Spritzflüssigkeiten, bei Standardflachstrahldüsen wird eine optimale Benetzung erreicht.

Die positiven Netz- und Hafteffekte gelten natürlich auch für andere Organismen und künstliche Oberflächen bzw. technische Anwendungen etwa zur Erreichung dünner Beschichtungen auf oder der Reinigung von Oberflächen.

Nachfolgend werden die Werte der dynamischen Oberflächenspannung (Krüss PocketDyne BP2100, T 24.6 °C) bei für das linearen C810 Glucamid und das Decenglucamid und ein Pelargonsäureamid gezeigt.

**Tabelle Dynamische Oberflächenspannung von Glucamiden (Temperatur 24.6 °C)**

| Substance | Conz [g/L] | Surface tension [mN/m] | | | |
|---|---|---|---|---|---|
| | | 20 ms | 50 ms | 100 ms | 200 ms |
| Synergen GA | 0.6 | 70.8 | 69.6 | 68.5 | 66.5 |
| Synergen GA | 1.25 | 66.2 | 62.5 | 60.3 | 58.1 |
| Synergen GA | 2.5 | 58.2 | 53.4 | 51.4 | 48.7 |
| Synergen GA | 5 | 45.2 | 41.5 | 39.8 | 38.0 |
| N-Methyl-N-dec-9-enoyl-glucamin | 0.6 | 70.2 | 68.9 | 67.6 | 67.4 |
| N-Methyl-N-dec-9-enoyl-glucamin | 1.25 | 66.4 | 63.7 | 61.8 | 61.0 |
| N-Methyl-N-dec-9-enoyl-glucamin | 2.5 | 59.7 | 56.6 | 55.5 | 54.1 |
| N-Methyl-N-dec-9-enoyl-glucamin | 5 | 50.5 | 48.3 | 47.7 | 46.6 |
| N-Methyl-N-nonanoyl-glucamin | 0.6 | 70.2 | 68.8 | 67.7 | 67.1 |
| N-Methyl-N-nonanoyl-glucamin | 1.25 | 66.0 | 63.7 | 62.6 | 61.1 |
| N-Methyl-N-nonanoyl-glucamin | 2.5 | 59.1 | 56.3 | 55.3 | 53.8 |
| N-Methyl-N-nonanoyl-glucamin | 5 | 47.7 | 45.6 | 45.2 | 44.3 |

Die Ergebnisse zur dynamischen Oberflächenspannung zeigen, dass das N-Methyl-N-dec-9-enoyl-glucamin ein vergleichbar geeignetes Netzmittel ist wie das Synergen GA (C810). Gleiches gilt für das Pelargonsäure-Glucamid. Beispiel 3 zeigt auch, dass das überraschende Ergebnis (Beispiel 2) der besseren Penetrationsförderung von Mesotrione durch das Decenglucamid im Vergleich zum Synergen GA (C810) nicht mit den Netzeigenschaften zusammenhängt.

### Beispiel 4

### Die Pflanzenverträglichkeit von N-Methyl-N-dec-9-enoyl-glucamin und

Pelargonsäure bei den oben genannten Konzentrationen von 0.6, 1.25, 2.5 und 5 g/l ist genauso gut wie die von Synergen GA und N-Methyl-N-nonanoyl-glucamin. Nach Applikation auf Poinsettia Pflanzen der Sorte Prinzetta gab es in keinem Fall Nekrosen oder andere Symptome, während ein mitgetesteter ethoxylierter Laurylalkohol bei 1 g/l innerhalb von einem Tag deutliche Nekrosen verursachte.

## Patentansprüche

1. Zuckertensid der Formel (I) 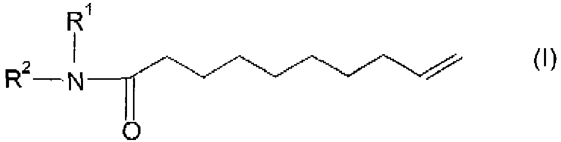 worin
R¹ H oder eine Alkylgruppe mit 1 bis 3 Kohlenstoffatomen bedeutet und
R² eine Polyhydroxy-hydrocarbyl-Gruppe mit einer linearen Hydrocarbylkette bedeutet, wobei mindestens drei Hydroxygruppen direkt an die Kette gebunden sind, bevorzugt eine 2,3,4,5,6-Pentahydroxyhex-1yl-Gruppe.

2. Zuckertensid gemäß Anspruch 1, **dadurch gekennzeichnet, dass** die Symbole in der Formel (I) folgende Bedeutungen haben:
R¹ ist eine Alkylgruppe mit 1 bis 3 Kohlenstoffatomen,
R² ist -CH₂-(CHOH)ₙ-CH₂OH, -CH₂-(CH₂OH)(CHOH)ₙ₋₁-CH₂OH, -CH₂-(CHOH)₂(CHOR³)(CHOH)-CH₂OH,
n ist 3, 4 oder 5 und
R³ ist H oder ein cyclisches Mono- oder Polysaccharid.

3. Zuckertensid gemäß Anspruch 1 oder 2, **dadurch gekennzeichnet, dass** die Symbole in der Formel (I) folgende Bedeutungen haben:
R¹ ist Methyl und
R² ist -CH₂-(CHOH)₄-CH₂OH.

4. Wässrige Adjuvant-Zusammensetzung enthaltend
A1) ein Zuckertensid der Formel (I) gemäß einem der Ansprüche 1 bis 3,
A2) Wasser,
A3) gegebenenfalls ein oder mehrere Co-Solventien,
A4) gegebenenfalls ein oder mehrere Ammoniumsalze und
A5) gegebenenfalls einen oder mehrere Enthärter.

5. Adjuvant-Zusammensetzung nach Anspruch 4, **dadurch gekennzeichnet, dass** in der Formel (I)
R¹ eine Methylgruppe und
R² CH₂-C(CHOH)₄-CH₂OH bedeutet.

6. Adjuvant-Zusammensetzung nach Anspruch 4 oder 5, **dadurch gekennzeichnet, dass** der Anteil des einen oder der mehreren Zuckertenside (I) 10 bis 90 Gew.-%, bevorzugt 20 bis 80 Gew.-% und besonders bevorzugt 30 bis 70 Gew.-%, bezogen auf das Gesamtgewicht der Zusammensetzung, beträgt.

7. Adjuvant-Zusammensetzung nach einem der Ansprüche 4 bis 6, **dadurch gekennzeichnet, dass** sie ein Co-Solvens (A3) enthält, gewählt aus Propylenglykol, Dipropylenglykol, Mischungen aus Propylenglykol und Dipropylenglykol, jeweils gegebenenfalls in Mischung mit Polypropylenglykol und/oder Polyethylenglykol, jeweils mit bis zu zehn Wiederholeinheiten, bevorzugt Propylenglykol.

8. Adjuvant-Zusammensetzung nach einem der Ansprüche 4 bis 7, **dadurch gekennzeichnet, dass** sie ein oder mehrere Ammoniumsalze (A4) enthält, bevorzugt aus der Gruppe bestehend aus Ammoniumsulfat, Ammoniumnitrat, Ammoniumnitratharnstoff, Ammoniumphosphat, Ammoniumcitrat, Ammoniumchlorid und Ammoniumthiosulfat
und/oder
dass sie einen oder mehrere Enthärter enthält, bevorzugt aus der Gruppe bestehend aus Alkaliphosphaten, Alkalicitraten, Alkalisulfaten, Glycerinsulfaten, Phosphonaten, Aminopolycarbonsäuren, Aminopolyphosphonsäuren, Gluconaten, Monocarbamid Dihydrogensulfat, Polycarboxylaten und Polyphosphonaten.

9. Verwendung der Adjuvant-Zusammensetzung nach einem oder mehreren der Ansprüche 4 bis 8 zur Steigerung der biologischen Aktivität von agrochemischen Wirkstoffen, vorzugsweise von Herbiziden, Pflanzennährstoffen und Pflanzenstärkungsmitteln.

10. Verwendung einer Adjuvant-Zusammensetzung nach einem oder mehreren der Ansprüche 4 bis 8 zur Herstellung einer wässrigen agrochemischen Wirkstoff-Zusammensetzung.

11. Wirkstoff-Zusammensetzung enthaltend
(W1) ein oder mehrere Zuckertenside der Formel (I) gemäß einem der Ansprüche 1 bis 3,
(W2) Wasser,
(W3) einen oder mehrere wasserlösliche Wirkstoffe,
(W4) gegebenenfalls mindestens ein Co-Solvens,
(W5) gegebenenfalls ein oder mehrere Ammoniumsalze,
(W6) gegebenenfalls einen oder mehrere Enthärter,
(W7) gegebenenfalls einen oder mehrere Hilfsstoffe und
(W8) gegebenenfalls einen oder mehrere wasserunlösliche Wirkstoffe.

12. Wirkstoff-Zusammensetzung gemäß Anspruch 11, **dadurch gekennzeichnet, dass** der mindestens eine wasserlösliche Wirkstoff ein agrochemischer Wirkstoff ist, bevorzugt aus der Gruppe bestehend aus Pestiziden, Pflanzennährstoffen und Pflanzenstimulantien.

13. Agrochemische Wirkstoff-Zusammensetzung nach Anspruch 12, **dadurch gekennzeichnet, dass** der eine oder die mehreren wasserlöslichen agrochemischen Wirkstoffe der Komponente (W3) ausgewählt sind aus der Gruppe der Pflanzennährstoffe, Pflanzenstärkungsmittel, Pflanzenwachstumsregulatoren und Herbizide, bevorzugt aus der Gruppe von Herbiziden bestehend aus den wasserlöslichen Salzen von Acifluorfen, Aminopyralid, Amitrol, Asulam, Benazolin, Bentazon, Bialaphos, Bispyribac, Bromacil, Bromoxynil, Bicyclopyron, Chloramben, Clopyralid, 2,4-D, 2,4-DB, Dicamba, Dichlorprop, Difenzoquat, Diquat, Endothal, Fenoxaprop, Flamprop, Florasulam, Flumiclorac, Fluoroglycofen, Fluroxypyr, Fomesafen, Fosamine, Glufosinat, Glyphosat, Imizameth, Imazamethabenz, Imazamox, Imazapic, Imazapyr, Imazaquin, Imazethapyr, MCPA, MCPB, Mecoprop, Mesotrione, Nicosulfuron, Octansäure, Pelargonsäure, Picloram, Quizalofop, 2,3,6-TBA, Sulcotrione, Tembotrione und Triclopyr.

14. Agrochemische Wirkstoff-Zusammensetzung nach Anspruch 12 oder 13, **dadurch gekennzeichnet, dass** das eine oder die mehreren wasserlöslichen agrochemischen Wirkstoffe der Komponente (W3) ausgewählt sind aus wasserlöslichen Salzen von 2,4-D, Bentazon, Dicamba, Fomesafen, Glyphosat, Glufosinat, MCPA, Mesotrione Paraquat und Sulcotrione, bevorzugt aus den wasserlöslichen Salzen von Glyphosat und Dicamba.

15. Agrochemische Wirkstoff-Zusammensetzung nach einem der Ansprüche 12 bis 14, **dadurch gekennzeichnet, dass** sie zusätzlich einen oder mehrere nichtwasserlösliche Wirkstoffe enthält.

16. Agrochemische Wirkstoff-Zusammensetzung nach einem der Ansprüche 12 bis 15, **dadurch gekennzeichnet, dass** das die Gesamtmenge der agrochemischen Wirkstoffe der Komponente (W3) in der Zusammensetzung größer als 100 g/l, bevorzugt größer als 200 g/l und besonders bevorzugt größer als 300 g/l, bezogen auf deren Säureäquivalent, beträgt.

17. Agrochemische Wirkstoff-Zusammensetzung nach einem oder mehreren der Ansprüche 12 bis 16, **dadurch gekennzeichnet, dass** die Gesamtmenge der Zuckertenside der Formel (I) in der Zusammensetzung von 20 bis 250 g/l, bevorzugt von 40 bis 200 g/l und besonders bevorzugt von 50 bis 150 g/l beträgt.

18. Agrochemische Wirkstoff-Zusammensetzung nach einem der Ansprüche 12 bis 17, **dadurch gekennzeichnet, dass** sie ein Ammoniumsalz (W5), bevorzugt gewählt aus der Gruppe bestehend aus Ammoniumsulfat, Ammoniumnitrat, Ammoniumnitratharnstoff, Ammoniumphosphat, Ammoniumcitrat, Ammoniumchlorid und Ammoniumthiosulfat enthält
und/oder
dass sie einen oder mehrere Enthärter enthält, bevorzugt aus der Gruppe bestehend aus Alkaliphosphaten, Alkalicitraten, Alkalisulfaten, Glycerinsulfaten, Phosphonaten, Aminopolycarbonsäuren, Aminopolyphosphonsäuren, Gluconaten, Monocarbamid Dihydrogensulfat, Polycarboxylaten und Polyphosphonaten.

19. Agrochemische Wirkstoff-Zusammensetzung nach einem oder mehreren der Ansprüche 12 bis 18, **dadurch gekennzeichnet, dass** sie neben der Komponente (W1) ein oder mehrere weitere Adjuvantien enthält.

20. Agrochemische Wirkstoff-Zusammensetzung nach einem der Ansprüche 12 bis 19, **dadurch gekennzeichnet, dass** sie als Konzentrat-Formulierung vorliegt, die vor dem Gebrauch verdünnt wird und 5 bis 80 Gew.-%, bevorzugt 10 bis 70 Gew. % und besonders bevorzugt 20 bis 60 Gew.-%, des einen oder der mehreren wasserlöslichen agrochemischen Wirkstoffe der Komponente (W3) und 1 bis 25 Gew.-%, bevorzugt 2 bis 20 Gew.-% und besonders bevorzugt 3 bis 15 Gew. %, des einen oder der mehreren Zuckertenside der Komponente (W1) enthält.

21. Agrochemische Wirkstoff-Zusammensetzung nach einem der Ansprüche 12 bis 20, **dadurch gekennzeichnet, dass** sie als Spritzbrühe vorliegt und 0,001 bis 10 Gew. %, bevorzugt 0,02 bis 3 Gew.-% und besonders bevorzugt 0,025 bis 2 Gew.-%, des einen oder der mehreren wasserlöslichen Pestizide der Komponente (W3) und 0,01 bis 1 Gew. %, bevorzugt 0,05 bis 0,5 Gew.-% und besonders bevorzugt 0,1 bis 0,25 Gew. %, der einen oder der mehreren Zuckertenside der Komponente (W1) enthält.

22. Verwendung einer agrochemischen Wirkstoff-Zusammensetzung nach einem der Ansprüche 12 bis 21 zur Kontrolle und/oder zur Bekämpfung von unerwünschtem Pflanzenwuchs, Pilzerkrankungen oder Insektenbefall bei Pflanzen, vorzugsweise zur Kontrolle und/oder zur Bekämpfung von unerwünschtem Pflanzenwuchs, wobei der agrochemische Wirkstoff ein Pestizid ist.

23. Verfahren zum Schutz von Pflanzen vor Schadorganismen, **dadurch gekennzeichnet, dass** man die Pflanze, die Schadorganismen oder deren Lebensraum mit einer agrochemischen Wirkstoff-Zusammensetzung, wobei der agrochemische Wirkstoff ein Pestizid ist, enthaltend eine erfindungsgemäße Adjuvant-Zusammensetzung nach einem der Ansprüche 4 bis 8, in Kontakt bringt.

## Claims

1. Sugar surfactant of the formula (I) 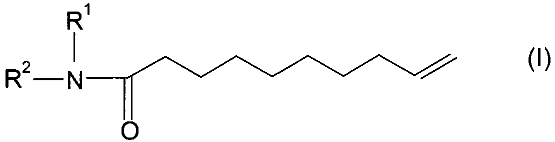 in which
R¹ is H or an alkyl group having 1 to 3 carbon atoms and
R² is a polyhydroxyhydrocarbyl group having a linear hydrocarbyl chain, where at least three hydroxyl groups are bonded directly to the chain, preferably a 2,3,4,5,6-pentahydroxyhex-1-yl group.

2. Sugar surfactant according to Claim 1, **characterized in that** the symbols in the formula (I) have the following definitions:
R¹ is an alkyl group having 1 to 3 carbon atoms,
R² is -CH₂-(CHOH)ₙ-CH₂OH, -CH₂-(CH₂OH)(CHOH)ₙ₋₁-CH₂OH, -CH₂-(CHOH)₂(CHOR³)(CHOH)-CH₂0H,
n is 3, 4 or 5 and
R³ is H or a cyclic mono- or polysaccharide.

3. Sugar surfactant according to Claim 1 or 2, **characterized in that** the symbols in the formula (I) have the following definitions:
R¹ is methyl and
R² is -CH₂-(CHOH)₄-CH₂OH.

4. Aqueous adjuvant composition comprising
A1) a sugar surfactant of the formula (I) according to any of Claims 1 to 3,
A2) water,
A3) optionally one or more cosolvents,
A4) optionally one or more ammonium salts and
A5) optionally one or more softeners.

5. Adjuvant composition according to Claim 4, **characterized in that** in formula (I)
R¹ is a methyl group and
R² is CH₂-C(CHOH)₄-CH₂OH.

6. Adjuvant composition according to Claim 4 or 5, **characterized in that** the proportion of the one or more sugar surfactants (I) is 10% to 90% by weight, preferably 20% to 80% by weight and more preferably 30% to 70% by weight, based on the total weight of the composition.

7. Adjuvant composition according to any of Claims 4 to 6, **characterized in that** it comprises a cosolvent (A3) selected from propylene glycol, dipropylene glycol, mixtures of propylene glycol and dipropylene glycol, each optionally in a mixture with polypropylene glycol and or polyethylene glycol, each having up to ten repeat units, preferably propylene glycol.

8. Adjuvant composition according to any of Claims 4 to 7, **characterized in that** it comprises one or more ammonium salts (A4), preferably from the group consisting of ammonium sulfate, ammonium nitrate, ammonium nitrate urea, ammonium phosphate, ammonium citrate, ammonium chloride and ammonium thiosulfate
and/or
**in that** it comprises one or more softeners, preferably from the group consisting of alkali metal phosphates, alkali metal citrates, alkali metal sulfates, glycerol sulfates, phosphonates, aminopolycarboxylic acids, aminopolyphosphonic acids, gluconates, monocarbamide dihydrogensulfate, polycarboxylates and polyphosphonates.

9. Use of the adjuvant composition according to one or more of Claims 4 to 8 for enhancement of the biological activity of active agrochemical ingredients, preferably of herbicides, plant nutrients and plant fortifiers.

10. Use of an adjuvant composition according to one or more of Claims 4 to 8 for production of an aqueous active agrochemical ingredient composition.

11. Active ingredient composition comprising
(W1) one or more sugar surfactants of the formula (I) according to any of Claims 1 to 3,
(W2) water,
(W3) one or more water-soluble active ingredients,
(W4) optionally at least one cosolvent,
(W5) optionally one or more ammonium salts,
(W6) optionally one or more softeners,
(W7) optionally one or more auxiliaries and
(W8) optionally one or more water-insoluble active ingredients.

12. Active ingredient composition according to Claim 11, **characterized in that** the at least one water-soluble active ingredient is an active agrochemical ingredient, preferably from the group consisting of pesticides, plant nutrients and plant stimulants.

13. Active agrochemical ingredient composition according to Claim 12, **characterized in that** the one or more water-soluble active agrochemical ingredients of component (W3) are selected from the group of the plant nutrients, plant fortifiers, plant growth regulators and herbicides, preferably from the group of herbicides consisting of the water-soluble salts of acifluorfen, aminopyralid, amitrole, asulam, benazolin, bentazone, bialaphos, bispyribac, bromacil, bromoxynil, bicyclopyrone, chloramben, clopyralid, 2,4-D, 2,4-DB, dicamba, dichlorprop, difenzoquat, diquat, endothal, fenoxaprop, flamprop, florasulam, flumiclorac, fluoroglycofen, fluroxypyr, fomesafen, fosamine, glufosinate, glyphosate, imizameth, imazamethabenz, imazamox, imazapic, imazapyr, imazaquin, imazethapyr, MCPA, MCPB, mecoprop, mesotrione, nicosulfuron, octanoic acid, pelargonic acid, picloram, quizalofop, 2,3,6-TBA, sulcotrione, tembotrione and triclopyr.

14. Active agrochemical ingredient composition according to Claim 12 or 13, **characterized in that** the one or more water-soluble active agrochemical ingredients of component (W3) are selected from water-soluble salts of 2,4-D, bentazon, dicamba, fomesafen, glyphosate, glufosinate, MCPA, mesotrione, paraquat and sulcotrione, preferably from the water-soluble salts of glyphosate and dicamba.

15. Active agrochemical ingredient composition according to any of Claims 12 to 14, **characterized in that** it additionally comprises one or more water-insoluble active ingredients.

16. Active agrochemical ingredient composition according to any of Claims 12 to 15, **characterized in that** the total amount of the active agrochemical ingredients of component (W3) in the composition is greater than 100 g/L, preferably greater than 200 g/L and more preferably greater than 300 g/L, based on the acid equivalent thereof.

17. Active agrochemical ingredient composition according to one or more of Claims 12 to 16, **characterized in that** the total amount of the sugar surfactants of the formula (I) in the composition is from 20 to 250 g/L, preferably from 40 to 200 g/L and more preferably from 50 to 150 g/L.

18. Active agrochemical ingredient composition according to any of Claims 12 to 17, **characterized in that** it comprises an ammonium salt (W5), preferably selected from the group consisting of ammonium sulfate, ammonium nitrate, ammonium nitrate urea, ammonium phosphate, ammonium citrate, ammonium chloride and ammonium thiosulfate
and/or
**in that** it comprises one or more softeners, preferably from the group consisting of alkali metal phosphates, alkali metal citrates, alkali metal sulfates, glycerol sulfates, phosphonates, aminopolycarboxylic acids, aminopolyphosphonic acids, gluconates, monocarbamide dihydrogensulfate, polycarboxylates and polyphosphonates.

19. Active agrochemical ingredient composition according to one or more of Claims 12 to 18, **characterized in that** it comprises, as well as component (W1), one or more further adjuvants.

20. Active agrochemical ingredient composition according to any of Claims 12 to 19, **characterized in that** it takes the form of a concentrate formulation which is diluted prior to use and contains 5% to 80% by weight, preferably 10% to 70% by weight and more preferably 20% to 60% by weight of the one or more water-soluble active agrochemical ingredients of component (W3) and 1% to 25% by weight, preferably 2% to 20% by weight and more preferably 3% to 15% by weight of the one or more sugar surfactants of component (W1).

21. Active agrochemical ingredient composition according to any of Claims 12 to 20, **characterized in that** it takes the form of a spray liquor and contains 0.001% to 10% by weight, preferably 0.02% to 3% by weight and more preferably 0.025% to 2% by weight of the one or more water-soluble pesticides of component (W3) and 0.01% to 1% by weight, preferably 0.05% to 0.5% by weight and more preferably 0.1% to 0.25% by weight of the one or more sugar surfactants of component (W1).

22. Use of an active agrochemical ingredient composition according to any of Claims 12 to 21 for control and/or for abatement of unwanted plant growth, fungal diseases or insect infestation in plants, preferably for control and/or for abatement of unwanted plant growth, the active agrochemical ingredient being a pesticide.

23. Method of protecting plants from harmful organisms, **characterized in that** the plant, the harmful organisms or their habitat is brought into contact with an active agrochemical ingredient composition, the active agrochemical ingredient being a pesticide, comprising an inventive adjuvant composition according to any of Claims 4 to 8.

## Revendications

1. Tensioactif à base de sucre de formule (I) 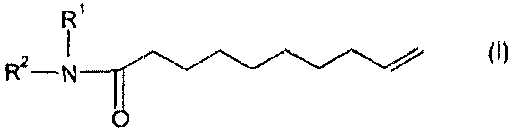 dans laquelle
R¹ signifie H ou un groupe alkyle comprenant 1 à 3 atomes de carbone et
R² signifie un groupe polyhydroxy-hydrocarbyle présentant une chaîne hydrocarbyle linéaire, au moins trois groupes hydroxy étant liés directement à la chaîne, de préférence un groupe 2,3,4,5,6-pentahydroxyhex-1-yle.

2. Tensioactif à base de sucre selon la revendication 1, **caractérisé en ce que** les symboles dans la formule (I) présentent les significations suivantes :
R¹ représente un groupe alkyle comprenant 1 à 3 atomes de carbone,
R² représente -CH₂-(CHOH)ₙ-CH₂OH, -CH₂-(CH₂OH)(CHOH)ₙ₋₁-CH₂OH, -CH₂-(CHOH)₂(CHOR³)(CHOH)-CH₂OH,
n vaut 3, 4 ou 5 et
R³ représente H ou un monosaccharide ou un polysaccharide cyclique.

3. Tensioactif à base de sucre selon la revendication 1 ou 2, **caractérisé en ce que** les symboles dans la formule (I) présentent les significations suivantes :
R¹ représente méthyle et
R² représente -CH₂-(CHOH)₄-CH₂OH.

4. Composition adjuvante aqueuse, contenant
A1) un tensioactif à base de sucre de formule (I) selon l'une quelconque des revendications 1 à 3,
A2) de l'eau,
A3) le cas échéant un ou plusieurs cosolvants,
A4) le cas échéant un ou plusieurs sels d'ammonium et
A5) le cas échéant un ou plusieurs adoucisseurs.

5. Composition adjuvante selon la revendication 4, **caractérisée en ce que**, dans la formule (I),
R¹ signifie un groupe méthyle et
R² signifie CH₂-C(CHOH)₄-CH₂OH.

6. Composition adjuvante selon la revendication 4 ou 5, **caractérisée en ce que** la proportion dudit un ou desdits plusieurs tensioactifs à base de sucre (I) représente 10 à 90% en poids, de préférence 20 à 80% en poids et de manière particulièrement préférée 30 à 70% en poids, par rapport au poids total de la composition.

7. Composition adjuvante selon l'une quelconque des revendications 4 à 6, **caractérisée en ce qu'**elle contient un cosolvant (A3) choisi parmi le propylèneglycol, le dipropylèneglycol, les mélanges de.propylèneglycol et de dipropylèneglycol, à chaque fois le cas échéant en mélange avec du polypropylèneglycol et/ou du polyéthylèneglycol, à chaque fois avec jusqu'à 10 unités récurrentes, de préférence le propylèneglycol.

8. Composition adjuvante selon l'une quelconque des revendications 4 à 7, **caractérisée en ce qu'**elle contient un ou plusieurs sels d'ammonium (A4), de préférence du groupe constitué par le sulfate d'ammonium, le nitrate d'ammonium, le nitrate d'ammonium-urée, le phosphate d'ammonium, le citrate d'ammonium, le chlorure d'ammonium et le thiosulfate d'ammonium
et/ou **en ce qu'**elle contient un ou plusieurs adoucisseurs, de préférence du groupe constitué par les phosphates de métal alcalin, les citrates de métal alcalin, les sulfates de métal alcalin, les sulfates de glycérol, les phosphonates, les acides aminopolycarboxyliques, les acides aminopolyphosphoniques, les gluconates, le dihydrogénosulfate de monocarbamide, les polycarboxylates et les polyphosphonates.

9. Utilisation de la composition adjuvante selon l'une ou plusieurs des revendications 4 à 8 pour l'augmentation de l'activité biologique de substances actives agrochimiques, de préférence d'herbicides, de substances nutritives pour plantes et d'agents de renforcement pour plantes.

10. Utilisation d'une composition adjuvante selon l'une ou plusieurs des revendications 4 à 8 pour la préparation d'une composition aqueuse de substances actives agrochimiques.

11. Composition de substances actives, contenant
(W1) un ou plusieurs tensioactifs à base de sucre de formule (I) selon l'une quelconque des revendications 1 à 3,
(W2) de l'eau,
(W3) une ou plusieurs substances actives solubles dans l'eau,
(W4) le cas échéant au moins un cosolvant,
(W5) le cas échéant un ou plusieurs sels d'ammonium,
(W6) le cas échéant un ou plusieurs adoucisseurs,
(W7) le cas échéant un ou plusieurs autres adjuvants et
(W8) le cas échéant une ou plusieurs substances actives insolubles dans l'eau.

12. Composition de substances actives selon la revendication 11, **caractérisée en ce que** ladite au moins une substance active soluble dans l'eau est une substance active agrochimique, de préférence du groupe constitué par les pesticides, les substances nutritives pour plantes et les stimulants pour plantes.

13. Composition de substances actives agrochimiques selon la revendication 12, **caractérisée en ce que** ladite une ou lesdites plusieurs substances actives agrochimiques solubles dans l'eau du composant (W3) sont choisies dans le groupe des substances nutritives pour plantes, des agents de renforcement pour plantes, des régulateurs de croissance pour plantes et des herbicides, de préférence dans le groupe des herbicides constitués par les sels solubles dans l'eau de l'acifluorfène, de l'aminopyralide, de l'amitrole, de l'asulame, de la bénazoline, de la bentazone, du bialaphos, du bispyribac, du bromacil, du bromoxynil, de la bicyclopyrone, du chlorambène, du clopyralid, du 2,4-D, du 2,4-DB, du dicamba, du dichlorprop, du difenzoquat, du diquat, de l'endothall, du fenoxaprop, du flamprop, du florasulam, du flumiclorac, du fluoroglycofène, du fluroxypyr, du fomesafen, de la fosamine, du glufosinate, du glyphosate, de l'imizameth, de l'imazaméthabenz, de l'imazamox, de l'imazapic, de l'imazapyre, de l'imazaquine, de l'imazéthapyre, du MCPA, du MCPB, du mécoprop, de la mésotrione, du nicosulfuron, de l'acide octanoïque, de l'acide pélargonique, du picloram, du quizalofop, du 2,3,6-TBA, de la sulcotrione, de la tembotrione et du triclopyr.

14. Composition de substances actives agrochimiques selon la revendication 12 ou 13, **caractérisée en ce que** ladite une ou lesdites plusieurs substances actives agrochimiques solubles dans l'eau du composant (W3) sont choisies parmi les sels solubles dans l'eau du 2,4-D, de la bentazone, du dicamba, du fomesafen, du glyphosate, du glufosinate, du MCPA, de la mésotrione, du paraquat et de la sulcotrione, de préférence parmi les sels solubles dans l'eau du glyphosate et du dicamba.

15. Composition de substances actives agrochimiques selon l'une quelconque des revendications 12 à 14, **caractérisée en ce qu'**elle contient en outre une ou plusieurs substances actives non solubles dans l'eau.

16. Composition de substances actives agrochimiques selon l'une quelconque des revendications 12 à 15, **caractérisée en ce que** la quantité totale des substances actives agrochimiques du composant (W3) dans la composition est supérieure à 100 g/l, de préférence supérieure à 200 g/l et de manière particulièrement préférée supérieure à 300 g/l, par rapport à leur équivalent d'acide.

17. Composition de substances actives agrochimiques selon l'une ou plusieurs des revendications 12 à 16, **caractérisée en ce que** la quantité totale des tensioactifs à base de sucre de formule (I) dans la composition est de 20 à 250 g/l, de préférence de 40 à 200 g/l et de manière particulièrement préférée de 50 à 150 g/l.

18. Composition de substances actives agrochimiques selon l'une quelconque des revendications 12 à 17, **caractérisée en ce qu'**elle contient un sel d'ammonium (W5), de préférence choisi dans le groupe constitué par le sulfate d'ammonium, le nitrate d'ammonium, le nitrate d'ammonium-urée, le phosphate d'ammonium, le citrate d'ammonium, le chlorure d'ammonium et le thiosulfate d'ammonium
et/ou **en ce qu'**elle contient un ou plusieurs adoucisseurs, de préférence du groupe constitué par les phosphates de métal alcalin, les citrates de métal alcalin, les sulfates de métal alcalin, les sulfates de glycérol, les phosphonates, les acides aminopolycarboxyliques, les acides aminopolyphosphoniques, les gluconates, le dihydrogénosulfate de monocarbamide, les polycarboxylates et les polyphosphonates.

19. Composition de substances actives agrochimiques selon l'une ou plusieurs des revendications 12 à 18, **caractérisée en ce qu'**elle contient, outre le composant (W1), un ou plusieurs autres adjuvants.

20. Composition de substances actives agrochimiques selon l'une quelconque des revendications 12 à 19, **caractérisée en ce qu'**elle se trouve sous forme de formulation de concentrat qui est diluée avant l'utilisation et qui contient 5 à 80% en poids, de préférence 10 à 70% en poids et de manière particulièrement préférée 20 à 60% en poids de ladite une ou desdites plusieurs substances actives agrochimiques solubles dans l'eau du composant (W3) et 1 à 25% en poids, de préférence 2 à 20% en poids et de manière particulièrement préférée 3 à 15% en poids dudit un ou desdits plusieurs tensioactifs à base de sucre du composant (W1).

21. Composition de substances actives agrochimiques selon l'une quelconque des revendications 12 à 20, **caractérisée en ce qu'**elle se trouve sous forme de bouillon de pulvérisation et contient 0,001 à 10% en poids, de préférence 0,02 à 3% en poids et de manière particulièrement préférée 0,025 à 2% en poids dudit un ou desdits plusieurs pesticides solubles dans l'eau du composant (W3) et 0,01 à 1% en poids, de préférence 0,05 à 0,5% en poids et de manière particulièrement préférée 0,1 à 0,25% en poids dudit un ou desdits plusieurs tensioactifs à base de sucre du composant (W1).

22. Utilisation d'une composition de substances actives agrochimiques selon l'une quelconque des revendications 12 à 21 pour la régulation de et/ou la lutte contre la croissance non souhaitée de plantes, la lutte contre les maladies fongiques ou l'attaque par les insectes sur des plantes, de préférence pour la régulation de et/ou la lutte contre la croissance non souhaitée de plantes, la substance active agrochimique étant un pesticide.

23. Procédé pour la protection de plantes contre des organismes nuisibles, **caractérisé en ce qu'**on met en contact la plante, les organismes nuisibles ou leur espace de vie avec une composition de substances actives agrochimiques, la substance active agrochimique étant un pesticide, contenant une composition adjuvante selon l'invention selon l'une quelconque des revendications 4 à 8.
